# EUROPEAN PATENT SPECIFICATION

(11) **EP 1 033 998 B1**
(45) Date of publication and mention of the grant of the patent: **19.10.2005**
(21) Application number: 98961894.7
(22) Date of filing: 03.12.1998
(51) Int. Cl.: A61K 38/17, A61P 25/28

(54) **SUPPRESSING BETA-AMYLOID-RELATED CHANGES IN ALZHEIMER'S DISEASE**
UNTERDRÜCKUNG VON VERÄNDERUNGEN IN ZUSAMMENHANG MIT BETA-AMYLOID BEI ALZHEIMER
SUPPRESSION DES MODIFICATIONS LIEES AUX BETA-AMYLOIDES DANS LA MALADIE D'ALZHEIMER

(30) Priority: 03.12.1997 US 67219 P; 27.03.1998 US 79697 P
(43) Date of publication of application: 13.09.2000
(73) Proprietor: Neuralab, Ltd., Smiths, FL04 (BM)
(72) Inventor: WEINER, Howard, L., Brookline, MA 02146 (US); SELKOE, Dennis, J., Jamaica Plain, MA 02130 (US)
(74) Representative: Mercer, Christopher Paul
(86) International application number: PCT/US1998/025694
(87) International publication number: WO 1999/027949

(56) References cited:
- WO-A-91/16819
- WO-A-95/31996
- WO-A1-96/39176
- TANAKA et al., "NC-1900, an Active Fragment Analog of Arginine Vasopressin, Improves Learning and Memory Deficits Induced by beta-Amyloid Protein in Rats", EUROPEAN JOURNAL OF PHARMACOLOGY, 1998, Vol. 352, pages 135-142, XP002916822
- GOZES et al., "Neuroprotective Strategy for Alzheimer Disease: Intranasal Administration of a Fatty Neuropeptide", PROC. NATL. ACAD. SCI. USA, January 1996, Vol. 93, pages 427-432, XP002916823
- FINCH et al., "Evolutionary Perspectives on Amyloid and Inflammatory Features of Alzheimer Disease", NEUROBIOLOGY OF AGING, 1996, Vol. 17, No. 5, pages 809-815, XP002916824
- TRIEB et al., "Is Alzheimer beta Amyloid Precursor Protein (APP) an Autoantigen? Peptides Corresponding to Parts of the APP Sequence Stimulate T Lymphocytes in Normals, But Not in Patients with Alzheimer's Disease", IMMUNOBIOLOGY, 1994, Vol. 191, No. 2-3, pages 114-115, Abstract C37, XP002916825

## Description

This application claims priority under 35 U.S.C. §119 of U.S. application serial no. 60/067,219, filed December 3, 1997 and U.S. application serial no. 60/079,697, filed March 27, 1998.

### Field of the Invention

This invention relates to a treatment for Alzheimer's disease based on mucosal tolerization.

### Background of the Invention

### 1. Alzheimer's Disease

Alzheimer's disease is the most common cause of cognitive degeneration in the elderly. Selkoe, D.J., *et al*., Science, **235,** 873-877 (1987). Extensive synaptic loss occurs with resulting progressive memory loss, psychological instability, decline in abstract reasoning, loss of bodily function, coma, and ultimately death. Extracellular lesions known as senile plaques develop in the brains of Alzheimer's victims, comprising insoluble aggregates of β-amyloid (Aβ) protein surrounded by dystrophic neurites. Mullan, M. and Crawford, F., Trends Neurosci., **16,** 398-403 (1993). β-Amyloid is a hydrophobic peptide comprising 39-43 amino acid residues; it is characterized by a high tendency to form aggregates, and results from proteolytic processing of a 90-140 kDa integral membrane protein precursor known as amyloid precursor protein (APP).

While formerly it was thought that β-amyloid peptide itself was responsible for neurodegeneration in Alzheimer's disease since the peptide can kill neurons in vitro, it is now known that the APP precursor is expressed in the brain cells of both healthy and Alzheimer's diseased brains. Konig, G., *et al*., Mol. Brain Res., **9,** 259-262 (1991). Hence, presence of the β-amyloid peptide is not by itself sufficient to account for the disease.

Attention has recently shifted to the potential involvement or participation in Alzheimer's of immunological processes such as inflammation. A large body of research evidence has accumulated showing certain parallels between immunological events and Alzheimer's disease. Alzheimer's brains display various structural features of immune-mediated brain damage including increased populations of microglial cells and astrocytes which express such inflammatory cytokines and inflammation-associated agents as interleukin-1 and α1-antichymotrypsin, and which are immunoreactive with major histocompatibility complex (MHC) class I and II. Abraham, *et al*., Cell, **52,** 487 (1988). Microglial cells are thought to be the functional equivalent of macrophages in the central nervous system. Stryen, *et al*., Exp. Neurol., **110,** 93 (1990). Other features observed in both immune-mediated and Alzheimer's brain damage include immunoreactive markers such as MHC class I and II glycoproteins, cytokines, complement receptors and regulatory factors. McGeer, P.L., *et al*., Neurosci. Lett., 107, 341-344 (1989); McGeer, P.L., *et al*., Brain Res., **544,** 315-320 (1990); Griffin, W.S., *et al*., Proc. Nat'l. Acad. Sci. U.S.A., **86,** 7611-7615 (1989). Also, the inflammatory mediator interleukin-6 (IL-6) which is found in Alzheimer's brains is known to stimulate α-2-macroglobulin (α2M), a highly potent human protease inhibitor produced by neurons of Alzheimer's brains, and thought to interfere with normal proteolytic processing of APP to form β-amyloid. Bauer, J., *et al*., FEBS Letts., **285,** 111-114 (1991). The physiological production of β-amyloid protein and mechanism of Alzheimer's disease is further described in Selkoe, Trends in Neurosciences, 16:403-409 (1993).

It is also known that β-amyloid peptide present in insoluble form in Alzheimer's brain plaques can bind and activate the classical complement proteins (C1q) *in vitro* in the absence of antibody proteins (e.g., immunoglobulins). In contrast, the soluble phase Aβ is subject to protective regulation by fluid-phase inhibitors, suggesting that it is the insoluble form of Aβ that is neurotoxic and explaining the apparent harmlessness of soluble Aβ. Rogers, *et al*., Proc. Nat'l Acad. Sci. U.S.A., **89,** 10016-10020 (1992). McGeer and Rogers and their collaborators have proposed that aggregated β-amyloid may activate the complement cascade which leads to the generation of proteins capable of destroying neurons. *Cf.* Schnabel, J., New Scientist, **138**, 22 (June 19, 1993). In further support of a mechanism link between amyloid plaque deposition and immune response, it was found that microglial cells, the immune cells of the brain, can be activated with interferon-γ (IFN-γ) and [1-42]Aβ to produce significantly more damage to neurons than either stimulus alone. Meda, L., *et al*., Nature, **374,** 647-650 (1995). This finding suggests a synergistic effect between Aβ and IFN-γ in triggering the generation by microglial cells of neurodegenerative substances such as reactive nitrogen intermediates (e.g., toxic free-radicals) and tumor necrosis factor-α (TNF-α). See, also Huberman, M. et al., J. Neuroimmunol., 52:147-152, 1994 who report increased secretion in Alzheimer's patients of proinflammatory cytokines, and a correlation between cytokine levels and stage of the disease.

WO 91/16819 and WO 95/31996 discuss administering low dosages of 10⁻¹⁰ mg to 10⁻² mg of β-amyloid protein for treatment of Alzheimer's disease and propose that such low dosages operate by a negative feedback mechanism in which administration of β-amyloid peptide prevents synthesis of further peptide.

Epidemiological evidence also supports a nexus between immunological activity and Alzheimer's disease. Anderson, K., *et al*., Neurol., 45, 1441-1445 (1995). In the Rotterdam study surveying 7,983 subjects, non-steroidal anti-inflammatory drugs were shown to have a protective effect on the risk for developing Alzheimer's disease and an ameliorating effect on the disease component of diminished cognitive function. In spite of these studies pointing to a role for the immune system in the induction of Alzheimer's disease, no truly effective therapeutic regimes have emerged based on these research results.

Current treatments for Alzheimer's disease are generally few and inadequate. TACRINE™ (1,2,3,4-tetrahydro-9-acridinamine monohydrochloride monohydrate; also marketed as COGNEX™) is a reversible cholinesterase inhibitor with some efficacy for treating Alzheimer's disease. Watkins, P.B., *et al*., J. Amer. Med. Assoc., **271,** 992 (1994). TACRINE has been only partially effective in community-dwelling subjects with mild or moderate dementia. Weiner, M.F., Consultant, **35,** 313 (1995). Aside from its limited efficacy, there are safety concerns for its use. These include the elevation of serum aminotransferase levels which may indicate hepatocellular injury. Other dangers involve a risk of developing liver necrosis, jaundice or hepatitis which can be severe or fatal in particularly susceptible patients.

Other approaches have been attempted, such as the treatment of psychological symptoms of Alzheimer's disease using selective serotonin re-uptake inhibitors, e.g., paroxetine, fluoxetine, or trazodone, and the administration of cholinergic agonists or precursors, e.g., lecithin and nicotine but have met with similarly limited success. Pilot clinical studies suggest that nicotine may be useful to treat deficits in attention and information processing resulting from Alzheimer's disease. Sahakian, *et al*., Brit. J. Psych., **154,** 797 (1989). Nicotine, however, is poorly bioavailable, and therefore not useful as a clinical treatment.

Viewing Alzheimer's disease as a chronic inflammatory state, McGeer and Rogers have proposed using anti-inflammatory agents such as indomethacin, a drug which curbs inflammation by blocking the action of cyclooxygenase, to treat the disease. McGeer, P.L., *et al*., U.S. Patent No. 5,192,753, issued March 9, 1993. In a study of 44 subjects with early-stage dementia, it was found that the group receiving the drug indomethacin did not deteriorate in mental function during the study. Schnabel, J., New Scientist, *supra.* However, various side effects of indomethacin, including gastro-intestinal effects, make its clinical use problematic.

Garvey, *et al*., U.S. Patent No. 5,409,946, issued April 25, 1995, disclose certain isoxazole, isothiazole and pyrazole compounds which allegedly enhance cognitive function, but do not appear to treat Alzheimer's disease directly.

In general, all known methods of treating Alzheimer's disease have met with no significant success. A clear need therefore exists for improved methods of treating the disease.

### 2. Oral and, More Generally, Mucosal Tolerization

A mucosal tolerization approach to treating T-cell mediated autoimmune diseases is discussed extensively in PCT International Application No. PCT/US93/01705. This method involves the oral or mucosal administration of antigens specific to the tissue under autoimmune attack which may or may not be themselves a target of the autoimmune attack ("bystander antigens"). Oral or mucosal administration of these "bystander antigens" causes active suppression, i.e. the induction of regulatory (suppressor) T-cells in the gut-associated lymphoid tissue (GALT), or, in the case of by-inhalation administration, bronchial-associated lymphoid tissue (BALT). See, also Miller, A., *et al*., J. Exp. Med., **174,** 791-798 (1991). These regulatory suppressor T-cells are released in the blood or lymphatic tissue and then migrate to the organ or tissue afflicted with the autoimmune disease where they suppress autoimmune attack mounted against the afflicted organ or tissue. The T-cells elicited by the bystander antigen (which recognize at least one epitope of the bystander antigen used to elicit them) are targeted to the locus of autoimmune attack where they mediate the local release of one or more immunomodulatory cytokines, such as transforming growth factor beta (TGF-β) interleukin-4 (IL-4) or interleukin-10 (IL-10) which down-regulate immune response.

Of these, TGF-β is an antigen-nonspecific immunosuppressive factor in that it suppresses all immune attack phenomena in the vicinity of its release regardless of the antigen that triggers these phenomena. (However, because oral tolerization with a bystander antigen causes the release of immunoregulatory substances only in the vicinity of autoimmune attack, no systemic immunosuppression ensues.)

IL-4 and IL-10 are also antigen-nonspecific immunoregulatory cytokines. IL-4 in particular enhances Th2 response, *i.e.*, it acts on T-cell precursors and causes them to differentiate preferentially into Th2 cells. IL-4 also indirectly inhibits Th1 exacerbation. IL-10 is a direct inhibitor of Th1 responses.

After mucosally tolerizing mammals afflicted with an autoimmune disease, increased levels of TGF-β, IL-4 and IL-10 were observed in the gut associated lymphoid tissue (Chen, Y., *et al*., Science, **265,** 1237-1240 (1994)) and a concomitant decrease in cytokines associated with immune system (and particularly T-cell activation), such as IL-2, IFN-γ, etc. Downregulation of inflammatory cytokines and upregulation of TGF-β, and IL-4 were observed in the brains of EAE rats following mucosal tolerization of those animals (Khoury *et al*., J. Exp. Med., **176,** 1355-1364 (1992)).

Naive T-cells differentiate into distinct populations defined by their cytokine secretion pattern and regulatory function. Th1 cells secrete predominantly interleukin-2 (IL-2) and IFN-γ and are involved in classic delayed-type hypersensitivity reactions, while Th2 cells, which secrete predominantly IL-4 and IL-10, induce selected immunoglobulin secretion and can down-regulate Th1-mediated immune responses Mossmann, T. R., *et al*., J. Immunol., **136,** 2348-57 (1986); Romagnan, S., Annu. Rev. Immunol., **12**, 227-57 (1994). Thus, cytokines secreted by T-cells after activation qualitatively influence the nature of the immune response. Recent reports have suggested that the dominant factors determining the maturation of naive T-cells into either predominantly Th1 or Th2 cells are cytokines, particularly IL-2 and IFN-γ for Th1 and IL-4 for Th2. Seder, R. A., *et al*., Annu. Rev. Immunol., **12,** 635-673 (1994). Other factors include the dose and type of antigen, the type of antigen-presenting cell (APC), and co-stimulatory molecules involved in activation. Once differentiated into Th1 or Th2 type, T-cells were formerly thought to be committed to the production of a given set of lymphokines upon restimulation. However, it has been recently discovered that certain MBP-specific, CD4⁺ T-cells, isolated from the GALT of SJL mice orally tolerized with myelin basic protein and MS patients orally tolerized with myelin and structurally identical to Th1 disease-inducing clones in terms of their T-cell receptor usage, MHC restriction and epitope recognition, suppressed rather than induced disease and may represent a novel T-cell subset (Th3) with both mucosal T-helper function and down-regulatory properties vis-a-vis Th1 and other immune cells.

T-cell secretion of certain cytokines is also implicated in the induction and regulation of autoimmune inflammatory disease, which is mediated by activated autoreactive T-cells that recognize self-tissue-specific antigen in the context of MHC class II molecules on APCs. In experimental autoimmune encephalomyelitis (EAE), a model for multiple sclerosis, it is believed that T-cell secretion of IL-2, IFN-γ, and TNF mediates inflammation and tissue damage, while the secretion of IL-4, IL-10, and TGF-β1 by myelin basic protein (MBP)-reactive T-cells is associated with potent suppressor activity and down-regulation of central nervous system inflammation. Miller, A., *et al*., Proc. Nat'l Acad. Sci. U.S.A., *supra* (1992). The induction of anti-inflammatory cytokines, including TGF-β1, appears to be of particular importance in regulating EAE. For example, anti-TGF-β1 monoclonal antibodies inhibit the suppressor effect of regulatory MBP-reactive T-cells. Chen, Y., *et al*., Science, *supra* (1994). Thus, autoreactive T-cells are not necessarily pathologic and can function to down-regulate immune responses associated with tissue inflammation locally. Similar findings have been made in humans afflicted with multiple sclerosis. TNF has been isolated from central nervous system plaques of MS patients, IFN-γ exacerbates attacks, and TGF-β and/or IL-4 and/or IL-10 is secreted by T-cells of MS patients tolerized with myelin.

### Objects of the Invention

It is an object of the present invention to provide novel immunomodulatory methods for treating individuals suffering from Alzheimer's disease and for preventing the disease in individuals at risk for contracting the disease.

Another object of the present invention is to provide compositions and pharmaceutical formulations useful for preventing the induction of Alzheimer's disease in animal models and for treating mammals suffering from the disease.

It is a further object of this invention to devise methods and compositions for suppressing certain cytokine responses associated with Alzheimer's disease in an individual by a mucosal mode of administration, and for enhancing certain other cytokine responses associated with the suppression of inflammatory responses associated with the disease.

It is another object of the invention to prevent deposition of β-amyloid.

These and other objects of the present invention will be apparent to those of ordinary skill in the art in light of the following.

### Summary of the Invention

It has been unexpectedly discovered by the present inventors that nasal administration in an aerosol form of an antigen associated with β-amyloid plaques (such as β-amyloid peptide and fragments thereof) is an effective treatment for disease associated with said plaques. In one aspect, the invention is directed to the use of an effective amount of an agent comprising an amino acid sequence of β-amyloid peptide or an effective fragment or analogue thereof in the manufacture of a medicament for nasal administration in an aerosol form, for treating a mammal suffering from Alzheimer's disease where the medicament is to be administered for a period of time sufficient to achieve at least one of the following:
(i) reduction in the level of one or more proinflammatory Th1 cytokines;
(ii) increase in the level of one or more anti-inflammatory Th2 or Th3 cytokines;
(iii) amelioration, inhibition or delay of the onset of at least one clinical symptom of Alzheimer's disease.
Nasally administered β-amyloid peptide according to the invention can initiate immunological responses in a subject which prevent, retard or arrest neuron cell destruction and/or prevent deposition of β-amyloid in neurons.

The present invention also relates to a pharmaceutical formulation adapted for nasal administration in an aerosol form to a mammal at risk of contracting Alzheimer's disease comprising an effective amount of a polypeptide comprising an amino acid sequence of β-amyloid peptide or an effective fragment or analog thereof for inhibiting or delaying the onset of at least one of the following:
(i) generation of one or more proinflammatory Th1 cytokines in the mammal;
(ii) decline in the quantity of one or more anti-inflammatory Th2 or Th3 cytokines in the mammal; or
(iii) appearance of at least one clinical symptom of the disease.
These and other embodiments of the subject invention will be described in the detailed description.

### Brief Description of the Drawings

Figure 1A illustrates proliferation (expressed as Stimulation Index (S.I.)) of T-cells from spleens of control and experimental SWISSXBDF1 mice either fed (75 µg/feeding x 5) or nasally treated (25 µg/application x 3) with [1-40]β-amyloid peptide and immunized two days after the last treatment with 100 µg [1-40]β-amyloid peptide in 50 µg CFA per mouse. Proliferation was determined ten days after immunization.
Figure 1B illustrates proliferation (expressed as S.I.) of popliteal lymph node T-cells from control and experimental SWISSXBDF1 mice and mice fed or nasally administered [1-40]β-amyloid peptide determined ten days after being immunized in the foot pad with 100 µg [1-40]β-amyloid peptide in 50 µg CFA per mouse. Control mice (designated "non") were only immunized.
Figure 2A illustrates proliferation (expressed as ΔCPM relative to nonimmunized mice) of T-cells from popliteal lymph nodes from SJL/J, DBA/1, C57BL/6, BALB/C and C3H/EB mice immunized with [1-40]β-amyloid peptide but not fed any peptide.
Figure 2B illustrates proliferation (expressed as ΔCPM as in Fig. 2A) of spleen T-cells from SJL/J, DBA/1, C57BL/6, BALB/C and C3H/EB mice immunized with [1-40]β-amyloid peptide but not fed any peptide.
Figure 3A illustrates the secretion (expressed as Δpg/ml relative to nonimmunized controls) of cytokines IL-2, IFN-γ, IL-4, IL-10 and TGF-β in popliteal lymph node T-cells from SJL/J, DBA/1, C57BL/6, BALB/C and C3H/EB mice immunized with [1-40]β-amyloid peptide but not fed any peptide.
Figure 3B illustrates the secretion (expressed in Δpg/ml as in Fig. 3A) of cytokines IL-2, IFN-γ, IL-4, IL-10 and TGF-β in T-cells isolated from spleens of SJL/J, DBA/1, C57BL/6, BALB/C and C3H/EB mice immunized with [1-40]β-amyloid peptide but not fed any peptide.
Figure 4A illustrates the secretion (expressed as in Fig. 3A) of cytokine IL-10 in T-cells from popliteal lymph nodes of C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 4B illustrates the secretion (expressed as in Fig. 3A) of cytokine IL-10 in T-cells from spleens of C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 5A illustrates the secretion (expressed as in Fig. 3A) of cytokine IL-2 in popliteal lymph node T-cells from C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 5B illustrates the secretion (expressed as in Fig. 3A) of TGF-β cytokine IL-2 in T-cells from spleens of C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 6A illustrates the secretion (expressed as in Fig. 3A) of TGF-β in popliteal lymph node T-cells from C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 6B illustrates the secretion (expressed as in Fig. 3A) of TGF-β in T-cells from spleens of C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA.
Figure 7A illustrates the secretion (expressed as in Fig. 3A) of IFN-γ from popliteal lymph node T-cells from SWISSXBDF1 mice fed 0 or 75 mg [1-40]β-amyloid peptide 5 times or nasally administered 25 mg of the same peptide 3 times.
Figure 7B illustrates the secretion (expressed as in Fig. 3A) of IL-2, IL-10, IL-4 and TGF-β from T-cells as described for Figure 7A.
Figure 8A illustrates the secretion (expressed as in Fig. 3A) of IFN-γ from T-cells as described in Fig. 6B.
Figure 8B illustrates the secretion of IL-2, IL-4, IL-10 and TGF-β from T-cells as described in Fig. 6B.
Figures 9A,9B, 9C, 9D list results from the assessment of plaque burden, cytokine secretion, and inflammatory markers based on examination of brain sections obtained from PDAPP mice that were orally or nasally administered [1-40] β-amyloid.
Figures 10A, 10B 10C show P values calculated using the 1-tailed Mann-Whitney U-test for various group comparisons among the PDAPP mice that were orally or nasally administered [1-40] β-amyloid.
Figure 11 shows results of immunoassays conducted on mouse antisera obtained from PDAPP mice to determine the presence of various subtypes of antibodies to β-amyloid.
Figure 12 is a bar graph showing results of *in vitro* experiments in which PLN from PDAPP mice that were immunized with [1-40] β-amyloid peptide prior to sacrifice were tested for production of IL-2.
Figure 13 is a bar graph showing results of *in vitro* experiments in which PLN from PDAPP mice that were immunized with [1-40] β-amyloid peptide prior to sacrifice were tested for proliferation in the presence of [1-40] β-amyloid peptide.
Figure 14 is a bar graph showing results of *in vitro* experiments in which PLN from PDAPP mice that were immunized with [1-40] β-amyloid peptide prior to sacrifice were tested for production of IFN-γ.
Figure 15 is a bar graph showing results of *in vitro* experiments in which PLN from PDAPP mice that were immunized with [1-40] β-amyloid peptide prior to sacrifice were tested for production of IL-6.
Figure 16 is a bar graph showing results of *in vitro* experiments in which PLN from PDAPP mice that were immunized with [1-40] β-amyloid peptide prior to sacrifice were tested for production of IL-10.
Figure 17 is a bar graph showing results of *in vitro* experiments in which PLN from PDAPP mice that were immunized with [1-40] β-amyloid peptide prior to sacrifice were tested for production of TGF-β.
Figure 18 shows results from an experiment in which [1-40] β-amyloid was nasally administered to (B6D2)F1 mice in conjunction with nasal administration of IL-4 or IL-10, and prior to immunization of the mice with [1-40] β-amyloid. The Figure shows proliferation *in vitro* of spleen cells from the mice in the presence of the [1-40] β-amyloid peptide.
Figure 19 shows results from an experiment in which [1-40] β-amyloid was nasally administered to (B6D2)F1 mice in conjunction with nasal administration of IL-4 or IL-10, and prior to immunization of the mice with [1-40] β-amyloid. The Figure shows production of IFN-γ *in vitro* in spleen cells from the mice in the presence of the [1-40] β-amyloid peptide.
Figure 20 shows results from an experiment in which [1-40] β-amyloid was nasally administered to (B6D2)F1 mice in conjunction with nasal administration of IL-4 or IL-10, and prior to immunization of the mice with [1-40] β-amyloid. The Figure shows production of IL-6 *in vitro* in spleen cells from the mice in the presence of the [1-40] β-amyloid peptide.
Figure 21 shows results from an experiment in which [1-40] β-amyloid was nasally administered to (B6D2)F1 mice in conjunction with nasal administration of IL-4 or IL-10, and prior to immunization of the mice with [1-40] β-amyloid. The Figure shows production of IL-10 *in vitro* in spleen cells from the mice in the presence of the [1-40] β-amyloid peptide.
Figure 22 shows results from an experiment in which [1-40] β-amyloid was nasally administered to (B6D2)F1 mice in conjunction with nasal administration of IL-4 or IL-10, and prior to immunization of the mice with [1-40] β-amyloid. The Figure shows production of TGF-β *in vitro* in spleen cells from the mice in the presence of the [1-40] β-amyloid peptide.

### Detailed Description of the Invention

The following terms as used in this disclosure have the meanings ascribed to them below.

"Mammal" is defined herein as any warm-blooded higher vertebrate organism (including a human) having an immune system and being susceptible to Alzheimer's disease or an induced or spontaneous animal model thereof.

"β-Amyloid peptide" is intended to include a peptide fragment of between about 10 to 43 (preferably 13-43) amino acid residues, the sequence of which is derived from β-amyloid precursor protein, prepared either by chemical synthetic methods known in the peptide art, including enzymatic methods or biosynthetic methods such as by isolation of peptide fragments from any source, including native tissue cultures, or by recombinant genetic engineering methods. Nonlimiting examples of β-amyloid peptide fragments encompassed by the present method and formulations include [1-40]Aβ, [1-42]Aβ, [25-35]Aβ, and analogs thereof containing substantially similar sequences and homologous, non-peptidic or isosteric replacements for selected residues. Effective peptide fragments include those having the property of suppressing a proinflammatory Th1 cytokine, decreasing the quantity of an inflammatory marker; increasing the quantity of an anti-inflammatory Th2 or Th3 cytokine, or suppressing β-amyloid deposition. Homologous replacements entail amino acid residues of related structure and differing only in the number of methylene groups present in the residue. Non-peptidic replacements include organic structures with conformational properties similar to those natural amino acid residues. Isosteric replacements refer to residues with either single atom substitutions (e.g., S for O in the aromatic hydroxyl in tyrosine) or substitutions of one moiety for another occupying a similar volume of space (e.g., an amino group for a methyl group). The useful β-amyloid peptides administered according to the invention include, among others, those having the sequences of human, bovine, and mouse β-amyloid. It is preferred to administer to humans a purified Aβ peptide having the amino acid sequence of the human or bovine Aβ peptide [1-43] or a fragment thereof recognized by the T-cells of the host that suppress autoimmune reaction associated with Alzheimer's disease. The peptide can form part of a longer amino acid sequence as long as the T-cell recognition site is not disturbed.

"Abatement", "suppression" or "reduction" of an immune response or reaction includes partial reduction or amelioration of one or more symptoms of the attack or reaction, *e.g.*, reduction in number of activated T-cells or in number of antibodies or in the levels of at least one proinflammatory cytokine (e.g. γ-IFN, IL-2, IL-6, or TNF) or an increase in the levels of at least one anti-inflammatory cytokine, such as TGF-β, IL-4, IL-10.

"Treatment" of Alzheimer's disease is intended to include, although not be limited to, one or more of the following:
(i) alteration of the profile of one or more cytokines or inflammatory markers in an Alzheimer's patient or animal model so that it conforms to or approaches the cytokine profile of a sex-and-age-matched subject without cognitive impairment;
(ii) a delay in the progress of Alzheimer's related dementia as evaluated by at least one of cognitive impairment and other NINCDS-ADRFA criteria (McKhan et al. 1984) including without limitation blood count, blood chemistry, Vitamin B12, Folic Acid, VDRL, thyroid function, electroencephalogram, computed tomography, or criteria according to the Record for Independent Living.
(iii) an amelioration of dementia as evaluated by one or more of the symptoms, factors and criteria detailed in item (ii) of this definition, or by observation by a physician specializing in Alzheimer's disease, or
(iv) a decrease in the amount of β-amyloid associated plaque than would otherwise have been observed absent the treatment.

Mucosal tolerance according to the invention is an advantageous method for treating Alzheimer's disease, dysfunction for at least the following reasons:
(1) Absence of toxicity. For example, no toxicity has been observed in clinical trials or animal experiments involving oral or other mucosal administration of other protein antigens, such as bovine myelin (which contains MBP and PLP) to humans afflicted with multiple sclerosis, or oral or by-inhalation administration of chicken Type II collagen to humans or rodents afflicted with rheumatoid arthritis (or a corresponding animal model disease); or oral administration of bovine S-antigen to humans afflicted with uveoretinitis; or oral administration of insulin to healthy volunteers.
(2) Convenience of therapy. Mucosal administration is more convenient than parenteral, or other forms, of administration.

The present inventors carried out a series of experiments to discover whether mucosal tolerization techniques causes suppression of β-amyloid-related inflammation characteristic of Alzheimer's disease. The use of the mucosal route to induce tolerance against an autoimmune response was found effective in an animal model to suppress undesirable immune responses associated with Alzheimer's disease, and to reduce histological symptoms of the disease in an animal model. Antigen feeding was implemented in test animals as a means of generating peripheral tolerance, and was successful in tolerizing Th1 type responses, while increasing the response of Th2 and Th3 lymphocytes, including increased anti-inflammatory cytokine responses as well as decreased popliteal lymph node proliferation in C3H/eb and DBA/1 mice. In a mouse model of Alzheimer's disease that simulates β-amyloid deposition, the PDAPP model, anti-inflammatory Th1 type responses were suppressed by mucosal administration of β-amyloid.

The present method of treating Alzheimer's disease based on the mucosal administration results, in one embodiment, in regulation of the specific immune response thought to be associated with the disease. While not wishing to be bound by theory, the present inventors believe that suppression of this response is effected primarily by an active suppression mechanism, i.e., the elicitation of T-cells characterized by an anti-inflammatory cytokine profile. Also, as discussed below, the method of the invention has been associated with induction of antibodies of β-amyloid which, without wishing to be bound by any theory of the invention, may have a role in the beneficial results observed.

As shown in the results described below, the present method can also result in suppression of the deposition of β-amyloid. The experiments described in the Examples show that β-amyloid deposition was suppressed in a mouse Alzheimer's disease model.

"Mucosal" administration includes oral, enteral, intragastric, nasal, buccal or intrapulmonary administration, and more generally any method of administration (e.g. by inhalation) of an active ingredient that brings the ingredient in contact with the immune system of the treated subject at the mucosa-associated lymphoid tissue, (MALT), including that of the gut, nasal, buccal, bronchial or pulmonary mucosa. In experiments described below, administration by inhalation has been found to be particularly effective.

In one embodiment, the present invention provides the use of an effective amount of a polypeptide comprising an amino acid sequence of β-amyloid peptide or an effective fragment or analog thereof in the manufacture of a medicament for nasal administration in an aerosol form for treating a mammal suffering from (or at risk for developing) Alzheimer's disease. The polypeptide may be, e.g. [1-40]β-amyloid, [1-42]β-amyloid, [25-35]β-amyloid or other β-amyloid peptide greater than about 10 amino acids in length. In one embodiment, the β-amyloid peptide sequence is greater than 15 amino acids in length, and in another greater than 20 amino acids in length. The medicament is to be administered for a period of time sufficient to achieve a change in one of the parameters described above. In a preferred embodiment, the present invention provides the use as described wherein the mammal is a human.

In additional embodiments, the present invention provides the pharmaceutical formulation as above-described further comprising a pharmaceutically acceptable carrier or diluent.

The present invention also provides a pharmaceutical formulation for administration to a mammal suffering from Alzheimer's disease comprising a dosage form according to the invention adapted for nasal administration. In one preferred embodiment, the present invention provides the pharmaceutical formulation in aerosol or spray form to be delivered by inhalation as described above further comprising a pharmaceutically acceptable carrier or diluent. The formulation can, for example, be administered in a nebulizer or inhaler. Such dosage forms are used to achieve the particularly beneficial results that have been determined by the inventors to be associated with administration by inhalation, e.g, by nasal administration.

### Antigens that May be Used to Induce Tolerance

Suitable antigens for use in the invention include antigens specific for Alzheimer's disease, i.e. antigens which are recognized by immune T-cells of a human or animal host. Non-limiting examples include synthetic or biosynthetic β-amyloid peptide, including peptide fragments thereof containing between 10 and 43 residues, preferably between 13 and 43 residues. Examples of such antigens include the β-amyloid peptides [1-40]Aβ, [1-42]Aβ and [25-35]Aβ. Encompassed within the scope of the present invention are various related substances sharing at least one such T-cell recognition site with β-amyloid peptide. Such substances include analogs of β-amyloid peptide which vary in length or composition, but containing substantially similar sequences (i.e., possessing significant levels, e.g., between about 40% and about 95%, of primary sequence homology). Such substances also include peptidic constructs of β-amyloid peptide (or aforementioned) analogs thereof and one or more flanking amino acid sequence found in native β-amyloid protein or of extra β-amyloid origin. The invention includes administration of amyloid precursor protein, and of homologs of that protein; examples are described in US Patent Nos. 5,525,714, 5,441,931, and 5,436,153. Amyloid precursor protein is also described in US Patent Nos. 5,318,958 and 5,218,100. cDNA clones encoding amyloid are also described in US Patent No. 4,912,206. The invention also encompasses peptides having conservative amino acid substitutions with respect to naturally occurring β-amyloid peptides, e.g., having one or more, preferably less than five, conservative substitutions with respect to the human, bovine, or mouse sequences. Methods for assessing the effectiveness of such β-amyloid peptides, β-amyloid peptide analogs and constructs sharing at least one T-cell recognition site with a β-amyloid peptide are well-known in the art. For example, the overlapping peptide method can be used to test various fragments (having at least 10 and preferably 20 amino acid residues) of β-amyloid peptide Aβ [1-39] (or Aβ [1-43] as the case may be) by placing them in contact with a preparation containing immune T-cells isolated from a host suffering from Alzheimer's disease and assessing the proliferation of such T-cells. Additionally, the cytokine patterns of such T-cells can be analyzed using the methodology provided below in the examples to further refine choice of a peptide. In one embodiment, the peptide is one which causes T-cells secreting anti-inflammatory cytokines to proliferate. In another embodiment, the peptide is one which causes suppression of one or more inflammatory responses, whether or not it causes T-cells secreting anti-inflammatory cytokines to proliferate or otherwise increases production of such anti-inflammatory cytokines. Peptides are also encompassed that suppress β-amyloid deposition, as shown, e.g., in the mouse model described in the Examples. In another embodiment, peptides are encompassed which cause an increase in the quantity of anti-β amyloid antibodies present in sera, e.g., in the sera of a human or non-human host suffering from, or predisposed to, Alzheimer's disease or a disease model of Alzheimer's disease. Peptides are also encompassed that have the sequence of a β-amyloid peptide, but including one, two, or three altered contact points for MHC or T-cell receptor, as determined by methods known in art using cells obtained from Alzheimer's patients.

### Formulations for Mucosal Administration

Administration of more than one antigen is possible, and may be desirable (e.g. when the patient's immune T-cells recognize more than one antigen).

The formulation according to the invention is a formulation adapted for nasal administration. The preparation of such a formulation is well within the skill of the art. It is preferred that the antigens employed be of synthetic provenance and not isolated from biological sources to avoid the risk of infection (notably, but not exclusively, to avoid transmission of agent responsible for the Creutzfeld-Jacob disease). Additionally, it is preferred that the formulation not contain adsorption promoting agents or ingredients that protect against proteolytic degradation. As further described below, however, the antigens can be combined with one ore more enhancing agents, such as IL-4 or IL-10, to increase the effectiveness of the formulation.

For by-inhalation administration (i.e. delivery to the bronchopulmonary mucosa) suitable sprays and aerosols can be used, for example using a nebulizer such as those described in U.S. Patent Nos. 4,624,251 issued November 25, 1986; 3,703,173 issued November 21, 1972; 3,561,444 issued February 9, 1971 and 4,635,627 issued January 13, 1971. The aerosol material is inhaled by the subject to be treated.

Other systems of aerosol delivery, such as the pressurized metered dose inhaler (MDI) and the dry powder inhaler as disclosed in Newman, S.P. in Aerosols and the Lung, Clarke, S.W. and Davia, D. eds. pp. 197-224, Butterworths, London, England, 1984, can be used when practicing the present invention.

Aerosol delivery systems of the type disclosed herein are available from numerous commercial sources including Fisons Corporation (Bedford, MA), Schering Corp. (Kenilworth, NJ) and American Pharmoseal Co. (Valencia, CA).

Formulations for nasal administration can be administered as a dry powder or in an aqueous solution. Preferred aerosol pharmaceutical formulations may comprise for example, a physiologically-acceptable buffered saline solution containing the β-amyloid antigen of the present invention.

Dry aerosol in the form of finely divided solid β-amyloid particles that are not dissolved or suspended in a liquid are useful in the practice of the present invention. The antigen may be in the form of dusting powders and comprise finely divided particles having an average particle size of between about 1 and 5 µm, preferably between 2 and 3 µm. Finely divided antigen particles may be prepared by pulverization and screen filtration using techniques well known in the art. The particles may be administered by inhaling a predetermined quantity of the finely divided material, which can be in the form of a powder.

Specific non-limiting examples of the carriers and/or diluents that are useful in the pharmaceutical formulations of the present invention include water and physiologically-acceptable buffered saline solutions such as phosphate buffered saline solutions pH 7.0-8.0.

The nasally administered formulation of the present invention may include a thermosetting gel which increases in viscosity at body temperature upon contact with the nasal mucosa.

Effective amounts are anticipated to vary according to the formulation employed. For formulation administered by inhalation, the effective amount is likely to be less than that of an oral dose.

The duration of treatment in humans should be a minimum of two weeks, and typically three months, and may be continued indefinitely or as long as benefits persist. The treatment may be discontinued if desired (in the judgment of the attending physician) and the patient monitored for signs of relapse. If clinical symptoms or other disease indicators show that the patient is relapsing, treatment may resume.

As will be understood by those skilled in the art, the dosage will vary with the antigen administered and may vary with the sex, age, and physical condition of the patient as well as with other concurrent treatments being administered. Consequently, adjustment and refinement of one or both of the dosages used and the administration schedules will preferably be determined based on these factors and especially on the patient's response to the treatment. Such determinations, however, require no more than routine experimentation, as illustrated in Examples provided below.

Administration of β-amyloid antigens can be conjoined with mucosal administration of one or more enhancers, i.e. substances that enhance one or more beneficial effect of the β-amyloid antigens. Such enhancers include LPS, Lipid A (as described in U.S. Application Serial No. 08/202,677, published as WO 91/01333) IL-4, IL-10 and Type I interferons, including α-interferon and β-interferon (See, e.g. U.S. Application Serial No. 08/420,980 and 08/420,979 and WO 95/27499 and WO 95/27500), cholera toxin B-subunit, and LTB (heat labile enterotoxin). Experiments have been carried out, described further below, in which administration of IL-4 in conjunction with β-amyloid peptide was found to be particularly effective. As used in this paragraph, "conjoined with" means before, substantially simultaneously with, or after administration of these antigens. Naturally, administration of the conjoined substance should not precede nor follow administration of the antigen by so long an interval of time that the relevant effects of the substance administered first have worn off. Therefore, enhancers should usually be administered within about 24 hours before or after the β-amyloid antigens and preferably within about one hour.

The invention is further described below by reference to examples, the purpose of which is to illustrate the present invention without limiting its scope.

### Example 1

### Experimental Animals

Female BALB/C (6-8 weeks of age); DBA/2; C3H/eb; C47b1/6; SWISSXBDF1 or SHL/J mice were used. The animals were maintained in a temperature- and light-controlled environment with free access to feed and water. Each experimental group contained no fewer than 5 mice.

### Immunization

Mice were immunized with 100 µg of [1-40]β-amyloid peptide ([1-40]Aβ) emulsified in 50 µg CFA (Difco Laboratories, Inc.). On day 10, popliteal lymph nodes and spleens were removed and for T-cell proliferation and cytokine production.

### Proliferation Assay

Cells (5 x 10⁵ per well) were cultured in triplicate in 96-well round-bottomed plates (Falcon™, Becton Dickinson, Lincoln Park, New Jersey) in the presence of antigen (1-100 µg/ml) for 72 hr. [³H]Thymidine (1 µCi/well) was added for the last 7 hr of culture before harvesting the cells. Incorporation of [³H]thymidine was determined using a Betaplate scintillation counter (Beckman Instruments Inc., Fullerton, California). The proliferation response was measured as Δcpm (cpm[test] - cpm[control without antigen]). The stimulation index (SI) was calculated as mean cpm[with antigen]/mean cpm[without antigen].

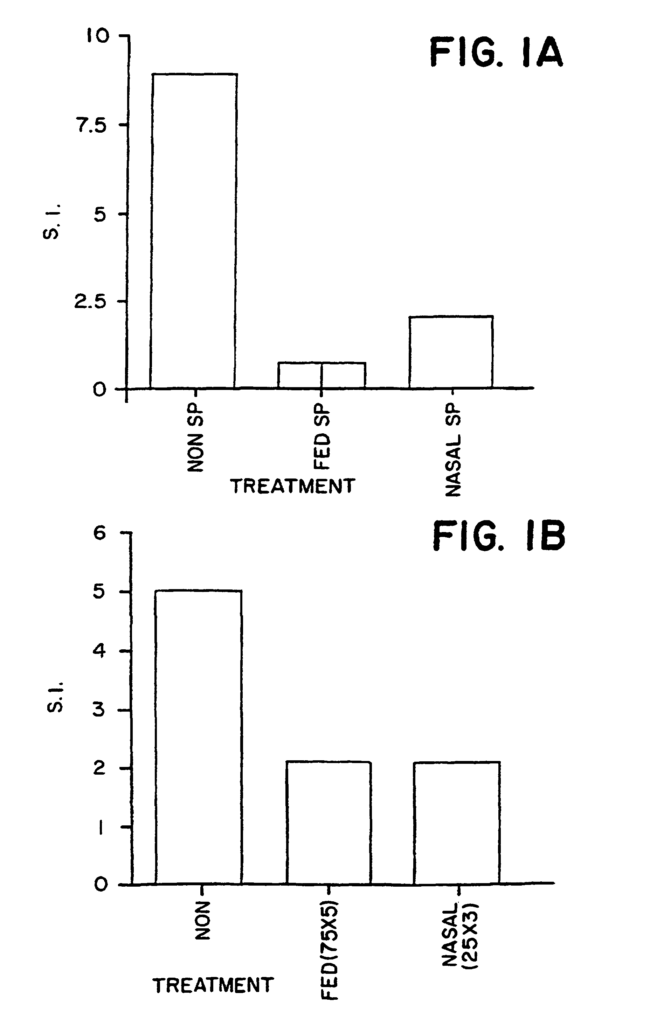

Figure 1A shows significant suppression of proliferation of spleen cells from SWISSXBDF1 mice (nontransgenic litermates of Alzheimer's model) either fed (75 µg/feeding x 5) or nasally treated (25 µg/direct application to nasal mucosa x 3) with [1-40]β-amyloid peptide and immunized two days after the last treatment with 100 µg [1-40]β-amyloid peptide in 50 µg CFA per mouse. Figure 1B shows suppression of proliferation in popliteal lymph nodes from SWISSXBDF1 mice either fed or nasally treated with [1-40]β-amyloid peptide determined ten days after being immunized in the foot pad with 100 µg [1-40]β-amyloid peptide in 50 µg CFA per mouse. The results show that proliferation of spleen and popliteal lymph node T-cells to [1-40] β-amyloid peptide was significantly suppressed in treated mice (fed or nasally administered the same peptide) compared to untreated controls. These results are particularly meaningful given that the cells tested for proliferative response were collected just 10 days after immunization, before the immunization effect subsided.

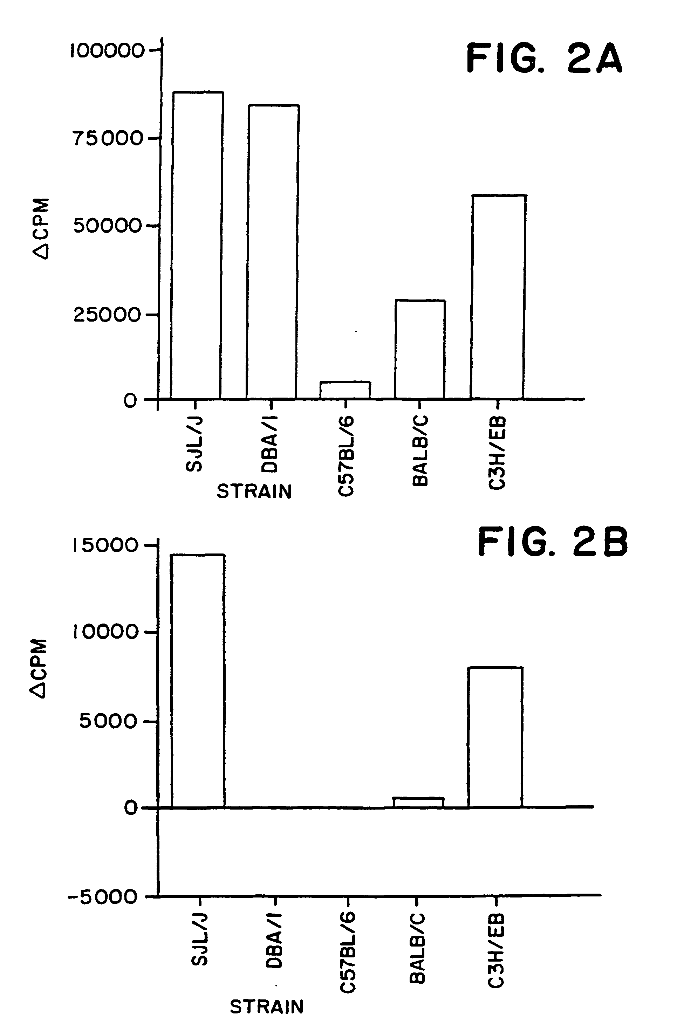

Figure 2A compares proliferation of popliteal lymph node T-cells from SJL/J, DBA/I, C57BL/6, BALB/C and C3H/EB mice immunized with [1-40]β-amyloid peptide. Figure 2B compares proliferation in spleen cells from SJL/J, DBA/1, C57BL/6, BALB/C and C3H/EB mice immunized with [1-40]β-amyloid peptide. This experiment shows that many strains of normal mice (not transgenic mice, not Alzheimer's model) respond to [1-40]β-amyloid peptide, within a short time after immunization. It should be noted that T-cells for the experiment shown in this Example are stimulated with antigen only once *in vitro* prior to the proliferation assay. Additional stimulations would enlist response from the apparent non-responder strain C57BL/6 and (DBA/1). This experiment provides information about the responding ability of cells from various strains and was useful in designing the subsequent experiments described below. It is believed to be advantageous to select "good responders" (such as C3H/EB) but not "too high responders" (SJL/J was thus not chosen).

### Example 2

### Cytokine Production Assays

Levels of IL-4, IL-2, IL-10, TGF-β and IFNγ in supernatants were determined by capture ELISA as described. Friedman, A., *et al*., Proc. Nat'l Acad. Sci. U.S.A., **91**, 6688 (1994); Yang, X., *et al*., J. Immunol., **150,** 4354 (1993). Briefly, supernatants were added to microtiter (Maxisorp) plates, previously coated with rat anti-mouse IL-4, IL-2, IL-10, TGF-β or IFN-γ monoclonal antibodies (capture antibodies, Pharmingen; 1 µg/ml at 4°C over night) and blocked with BSA-diluent/blocking solution (KPL). Plates were washed, and standards and samples were added for another overnight incubation at 4°C. Bound cytokine was detected by the addition of ABTS (Kirkegaard & Perry). Wells were washed, and cytokine levels determined using biotinylated rat anti-mouse IL-4, IL-2, IL-10, TGF-β or IFN-γ monoclonal antibodies (detecting antibodies, Pharmingen) and a peroxide visualization system (peroxidase-labeled streptavidin). IL-2 and IL-4 were analyzed after 24 hours of cell incubation with antigen, IL-10 and IFN-γ after 40 hours and TGF-β after 72 hours. Cytokine levels were calculated from a log-log plot of absorbance vs. concentration of recombinant cytokines (Pharmingen), and results are expressed in pg/mL (for IL-4, IL-2, IL-10, TGF-β and IFN-γ). Threshold sensitivities of ELISA assays were 5 pg/mL, 10 pg/mL and 2.5 ng/mL for IL-4, IL-2 and IFN-γ, respectively.

### Statistical analysis

The statistical significance of differences between experimental groups was determined using unpaired two-tailed Student's t-test, with differences considered significant at P < 0.05.

### Determination that mice exposed to Aβ peptide either fed or nasally administered display suppression of immunostimulatory (proinflammatory) cytokines (e.g. IL-2 and IFN-γ) and enhancement of immunoregulatory (anti-inflammatory) cytokines (e.g. IL-4, IL-10 and TGF-β) in vivo.

C3H/eb mice were fed [1-40]Aβ 500 µg/feeding, 50 µg/feeding, or 5 µg/feeding every day for 5 days (200 µl/feeding). Nasal administration was given at 50 µg/application or 5 µg/application every day for 3 days (10 µl/application). Two days after the last feeding or nasal administration, fed, nasal or non-fed mice were immunized in the foot pad with 100 µg [1-40]Aβ in 50 µg CFA per mouse.

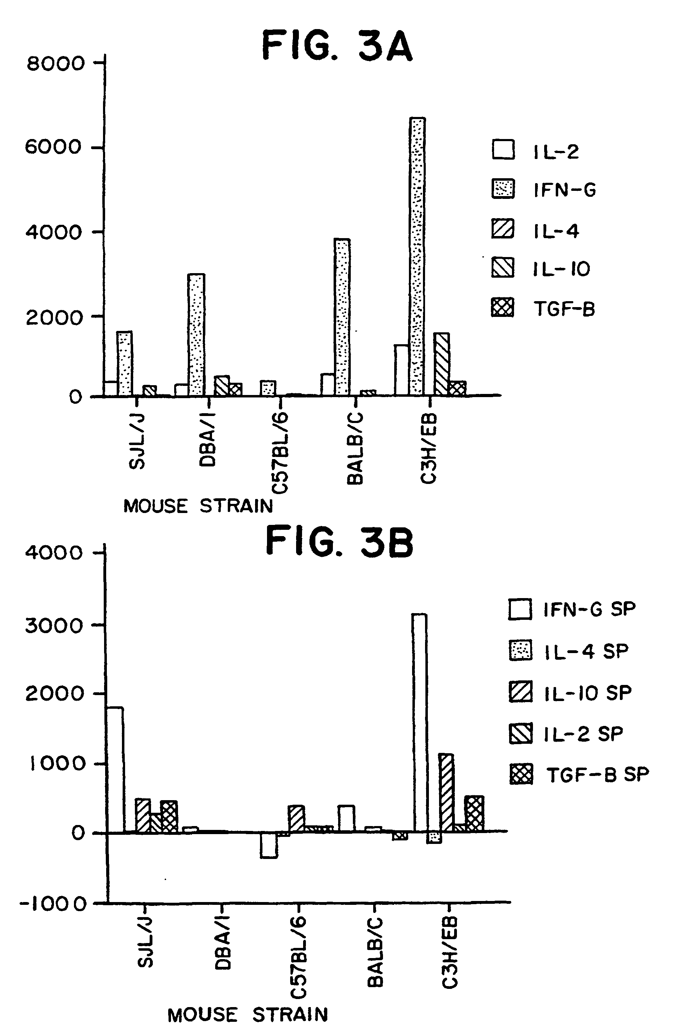

Figure 3A provides a comparison of the secretion of cytokines IL-2, IFN-γ, IL-4, IL-10 and TGF-β in popliteal lymph nodes from SJL/J, DBA/1, C57BL/6, BALB/C and C3H/EB mice immunized with [1-40]β-amyloid peptide but not fed or otherwise treated with the peptide. Figure 3B compares the secretion of these cytokines in spleen cells from a similar range of mice strains immunized with [1-40]β-amyloid peptide (also not treated with the peptide) Comparison of the results in Fig. 3A and Fig. 3B shows that the T-cells from spleens secrete a different cytokine profile from that of T-cells from popliteal lymph nodes, and that the cytokine profile varies with the strain of mouse. Again, the reason for this experiment is to select responders that will show a difference in the property tested under the experimental conditions.

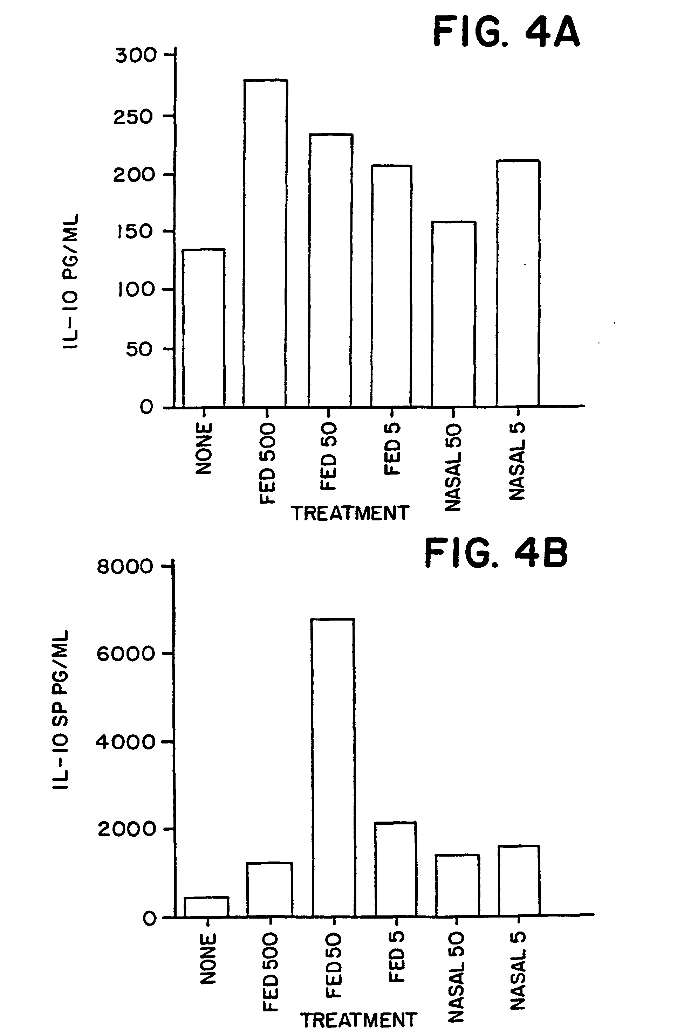

Figure 4A shows the secretion of the immunoregulatory cytokine IL-10 in popliteal lymph nodes from C3H/EB mice either fed (5, 50 or 500 µg/feeding for 5 days) or nasally administered (5 or 50 µg/application for 3 days) [1-40]β-amyloid peptide prior to immunization (2 days after the last feeding or nasal application) with 100 µg [1-40]β-amyloid peptide in 50 µg CFA. Figure 4B demonstrates the secretion of cytokine IL-10 in spleen cells from C3H/EB mice either fed or nasally administered [1-40]β-amyloid peptide under similar conditions. This experiment also served as a preliminary dose response experiment for this mouse strain. Given that the T-cells underwent a single stimulation *in vitro* and that the effects of the immunization (which would suppress IL-10 levels in all mice tested) had not subsided, the results, which show significant enhancement of IL-10 in at least one of spleen and PLN cells treated with at least one dose, provides a showing that the cytokine profile of T-cells that recognize [1-40]β amyloid peptide can be altered by tolerization to the same antigen.

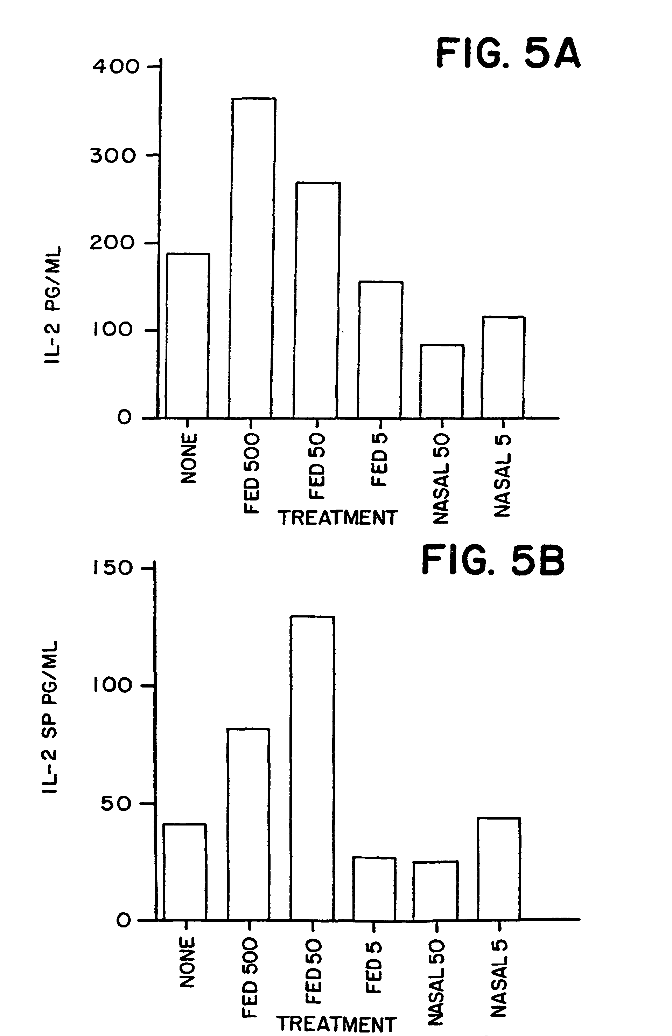

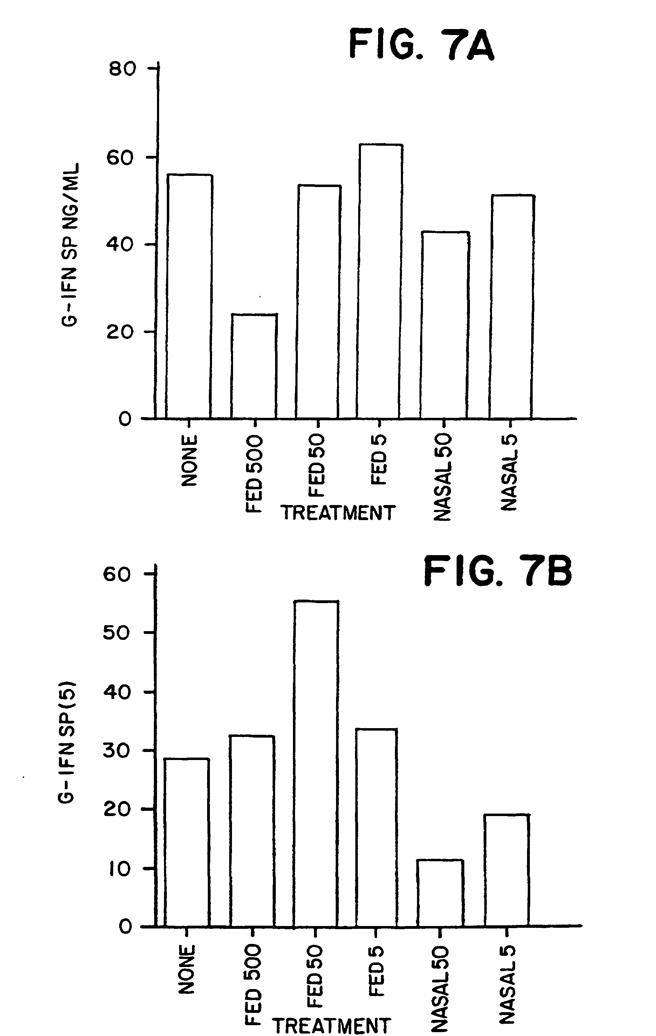

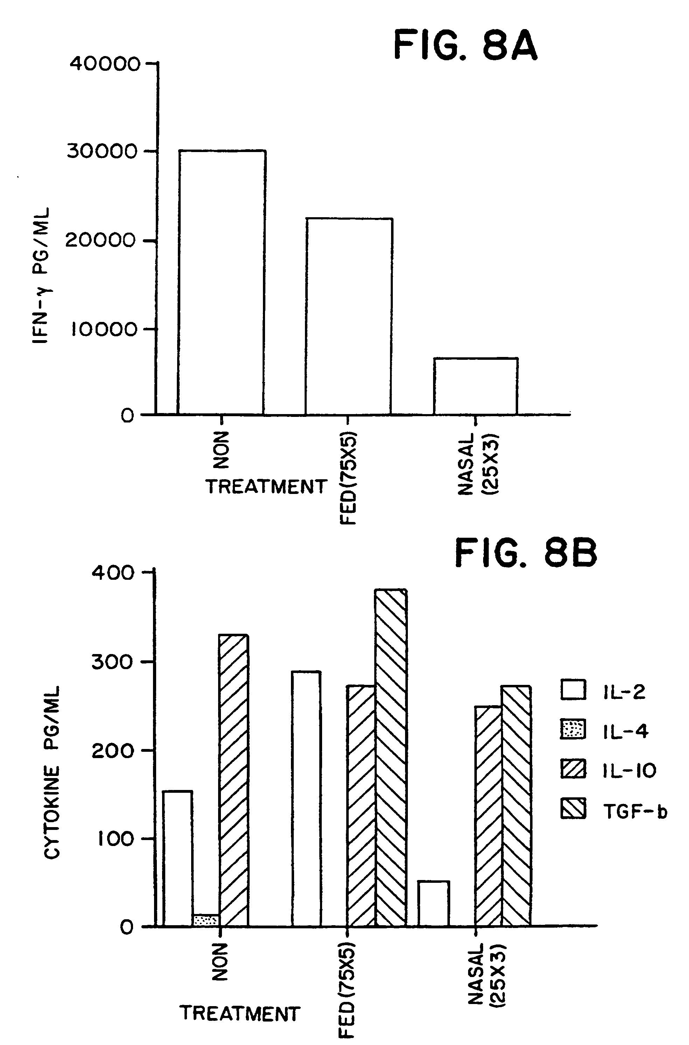

Figure 5A illustrates the result in secretion of the Th1 immunostimulatory (proinflammatory) cytokine IL-2 in popliteal lymph nodes from C3H/EB mice at various feeding levels (5, 50 and 500 µg/feeding for 5 days) and when nasally administered (5 or 50 µg/application for 3 days) [1-40]β-amyloid peptide prior to immunization (2 days after the last feeding or nasal application) with 100 µg [1-40]β-amyloid peptide in 50 µg CFA. Figure 5B similarly shows the secretion of cytokine IL-2 in spleen cells from C3H/EB mice either fed (at lower doses, e.g., 5 µg/feeding for 5 days) or nasally administered (at 50 µg/application for 3 days) [1-40]β-amyloid peptide prior to immunization (2 days after the last feeding or nasal application) with 100 µg [1-40]β-amyloid peptide in 50 µg CFA. The fact that IL-2 secretion is suppressed at least at one dose of treatment in either the spleen or the PLN T-cells shows suppression of Th1 responses despite the nonoptimal (i) timing, of the experiment (relative to the time of immunization); (ii) amount fed; and (iii) stimulation of cells *in vitro* (only once). Suppression of IL-2 secretion was clear by nasal administration (Fig. 5A) and at 5 µg fed and 50 µg nasal (Fig. 5B). Based on the results for Fig. 5A, and Figs. 5B, 6A, 6B, and 7A, 7B, it was decided to use 25 µg nasally and 75 µg orally in subsequent experiments. These are not optimal amounts, but they provide a quantity for demonstrating differences within the experimental conditions employed. See Figs. 8A and 8B below.

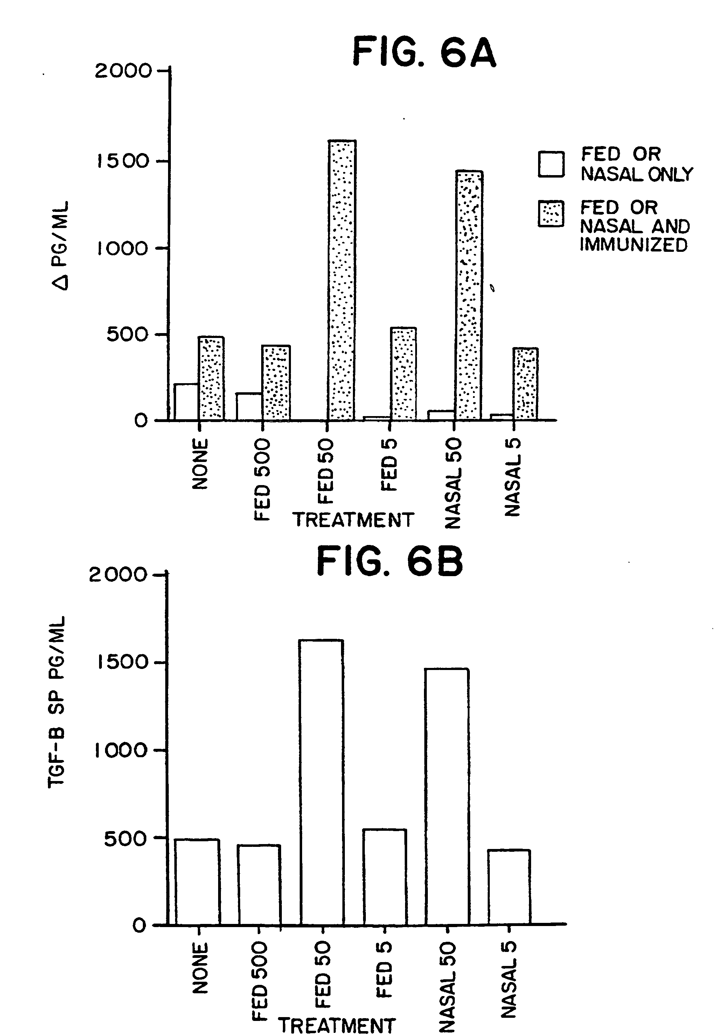

Figure 6A shows secretion of TGF-β in popliteal lymph nodes from C3H/EB mice either fed (at lower doses; 5 or 50 µg/feeding for 5 days) or nasally administered (at 50 µg/application for 3 days) [1-40]β-amyloid peptide prior to immunization (2 days after the last feeding or nasal application) with 100 µg [1-40]β-amyloid peptide in 50 µg CFA. Figure 6B illustrates the enhanced secretion of TGF-β in spleen cells from C3H/EB mice either fed (at lower dose; 5 and 50 µg/feeding for 5 days) or nasally administered (at 50 µg/application for 3 days) [1-40]β-amyloid peptide prior to immunization (2 days after the last feeding or nasal application) with 100 µg [1-40]β-amyloid peptide in 50 µg CFA.

### Example 3

### Cytokine profiles after feeding or nasal administration and immunization of non-transgenic litermates of the transgenic mouse model of Alzheimer's disease.

**Feeding and Immunization:** SWISSXXB6D2F1 mice were fed with [1-40]Aβ 75 µg/feeding x 5 or nasally administered with 25 µg/application x 3, and 2 days after the last treatment, were immunized with 100 µg [1-40]Aβ in 50 µg CFA. Ten days later, the popliteal lymph node and spleen T-cells were checked for cytokine production as described above. The results of these experiments demonstrated that mice pretreated with Aβ peptide and immunized with Aβ peptide had a reduced *in vitro* response to Aβ stimulation for proinflammatory (Th1) cytokines (and IFN-γ and IL-2 at least pursuant to nasal administration of 25 µg) and an increased response for anti-inflammatory (Th2) cytokines (TGF-β and IL-10). The absence of detected TGF-β in non-treated mice in Figure 8B is particularly significant. These observations confirmed studies disclosed in Examples 1-2 on normal mice of several strains showing increased anti-inflammatory cytokine responses as well as decreased PLN proliferation in mice of certain strains (e.g., C3H/EB and DBA/1) which had been mucosally tolerized with Aβ peptide.

### Example 4

### Feeding, nasal administration and immunization of heterozygous PDAPP transgenic mice (animal model of Alzheimer's disease).

Transgenic PDAPP mice were acquired from Athena Neurosciences, Inc. (South San Francisco, CA). These mice express a human gene encoding a mutant form of β-amyloid precursor protein and progressively develop β-amyloid deposits in the hippocampus and cerebral cortex in a manner similar to that seen in Alzheimer's disease. The β-amyloid deposits in the transgenic mice become associated with activated microglial cells, reactive astrocytes and other features of the periplaque inflammatory response seen in Alzheimer's disease brains.

4 to 5 month old PDAPP mice were fed or nasally administered either [1-40]β-amyloid peptide or a control protein (myelin basic protein or ovalbumin in amounts of 50 or 500 µg per feeding). Administration was carried out three (nasal) or five times consecutively (oral) during an initial one week period, and then on a weekly basis until the animals were between 11 and 12 months old. The orally administered β-amyloid peptide was in a dosage of 10 or 100 µg, and the nasally administered β-amyloid was in a dosage of 5 or 25 µg.

The feeding and nasal administration of β-amyloid peptide, or ovalbumin to PDAPP mice was conducted among the following mice groups:
- Group 0:: Non treated; mice nos. 0.1-0.7
- Group 1:: fed [1-40]β-amyloid peptide 100µg/feeding; mice nos. 1.1-1.9
- Group 2:: fed [1-40]β-amyloid peptide 10µg/feeding; mice nos. 2.1-2.9
- Group 3:: nasal [1-40]β-amyloid peptide 25µg/treatment; mice nos. 3.1-3.9
- Group 4:: nasal [1-40]β-amyloid peptide 5µg/treatment; mice nos. 4.1-4.7
- Group 5:: fed ovalbumin 500µg/feeding; mice nos. 5.1-5.6
- Group 6:: nasal ovalbumin 50µg/treatment; mice nos. 6.1-6.6
- Group 7:: fed MBP 500µg/feeding; mice nos. 7.1-7.5
- Group 8:: nasal MBP 50µg/treatment; mice nos. 8.1-8.6

Two mice from each of groups 1, 5, and 8 were immunized with β-amyloid peptide 10 days prior to sacrifice.

At the end of the treatment period, the mice were sacrificed and brains, popliteal lymph nodes and spleens removed. Brains were removed and immersed in 70% ethanol fixative following by paraffin embedding and sectioning. One hemisphere was frozen and cryostat-sectioned.

Thereafter, sections of the brains of the orally treated mice were examined using antibodies and histological stains. Antibodies to glial fibrillary acidic protein (GFAP) were employed to show abnormal astrocytes, and antibody to amyloid precursor protein employed to show abnormal neurites. Lymphocytes were examined for cytokine production *in vitro* in the presence of β-amyloid.

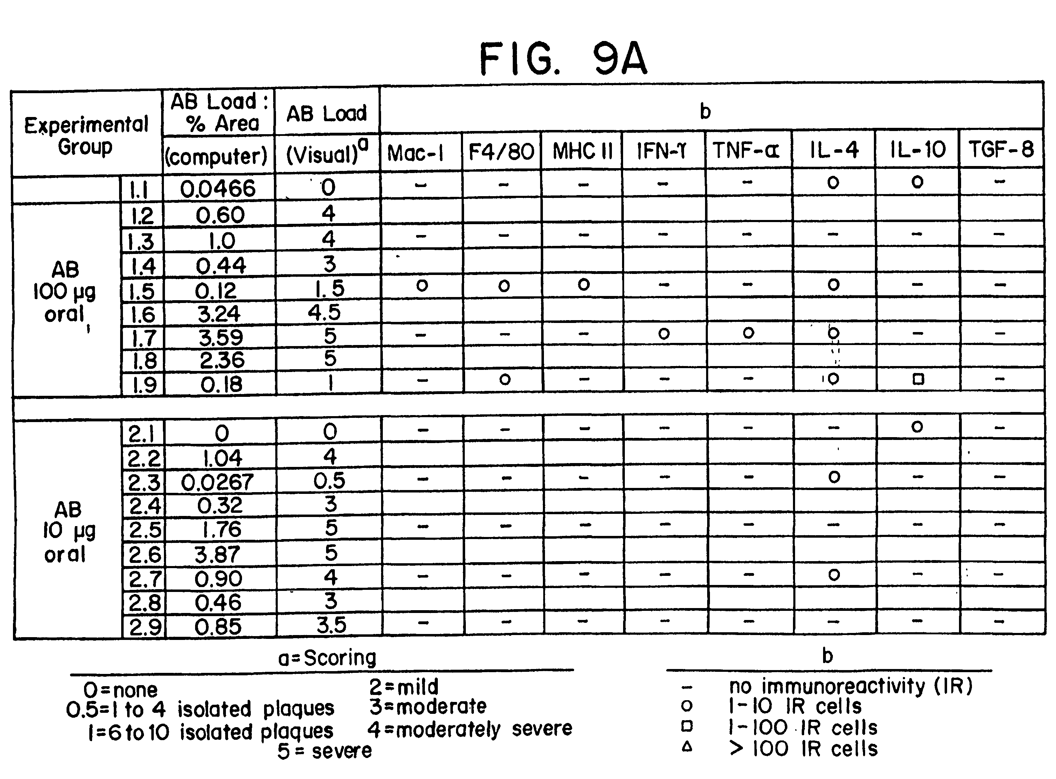

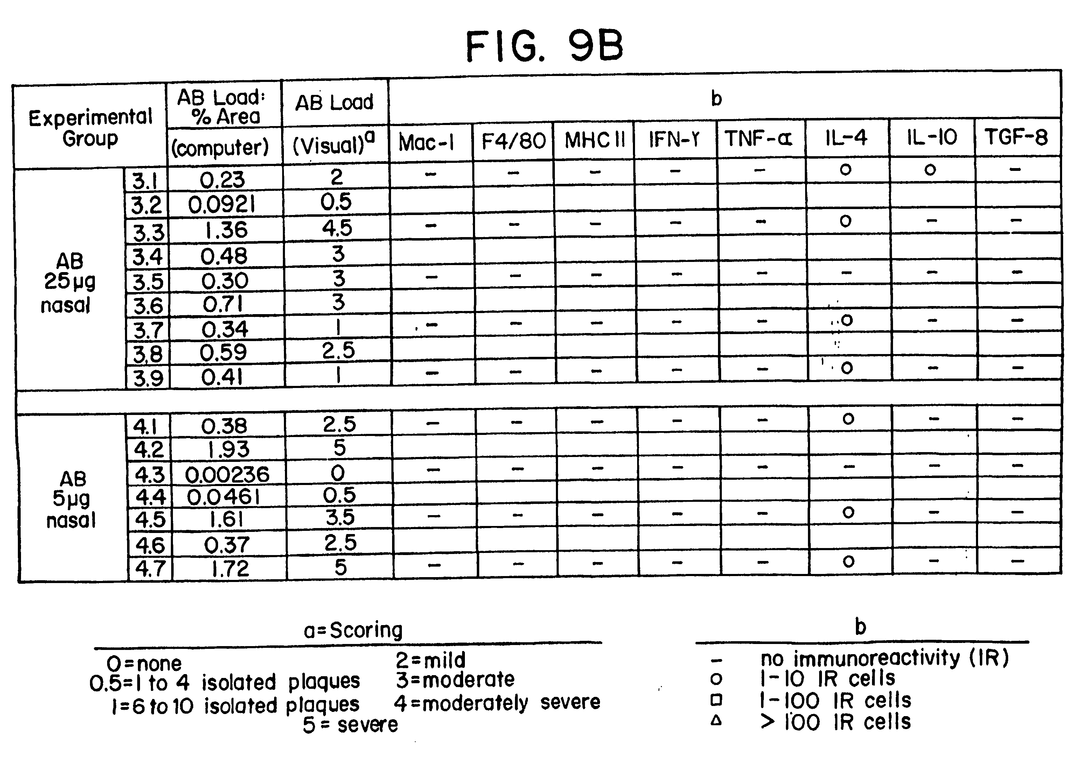

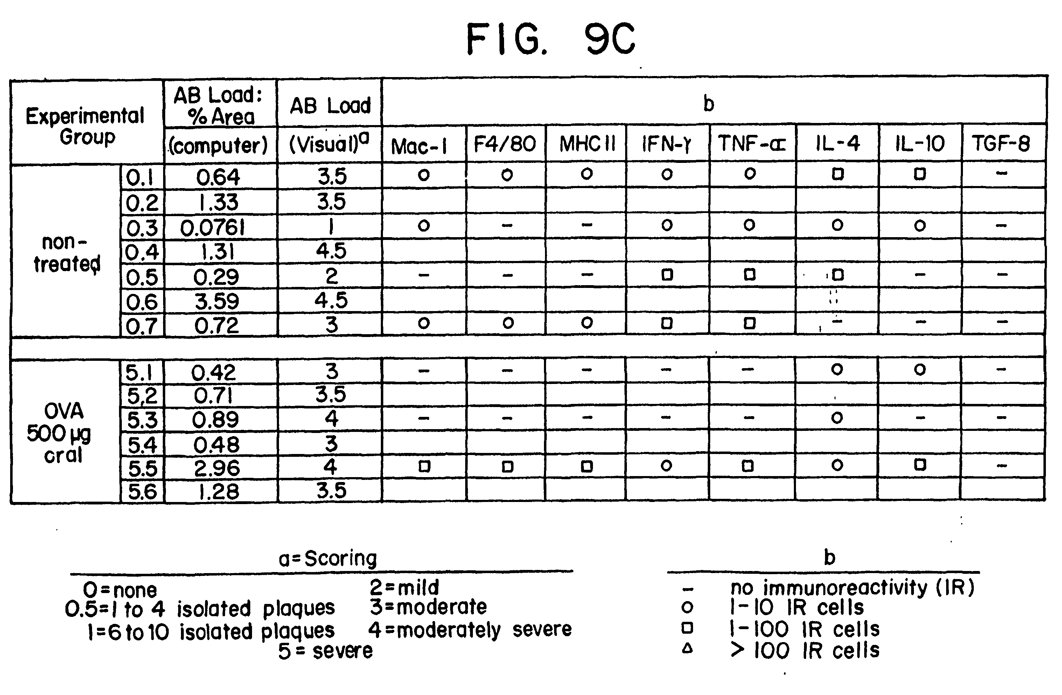

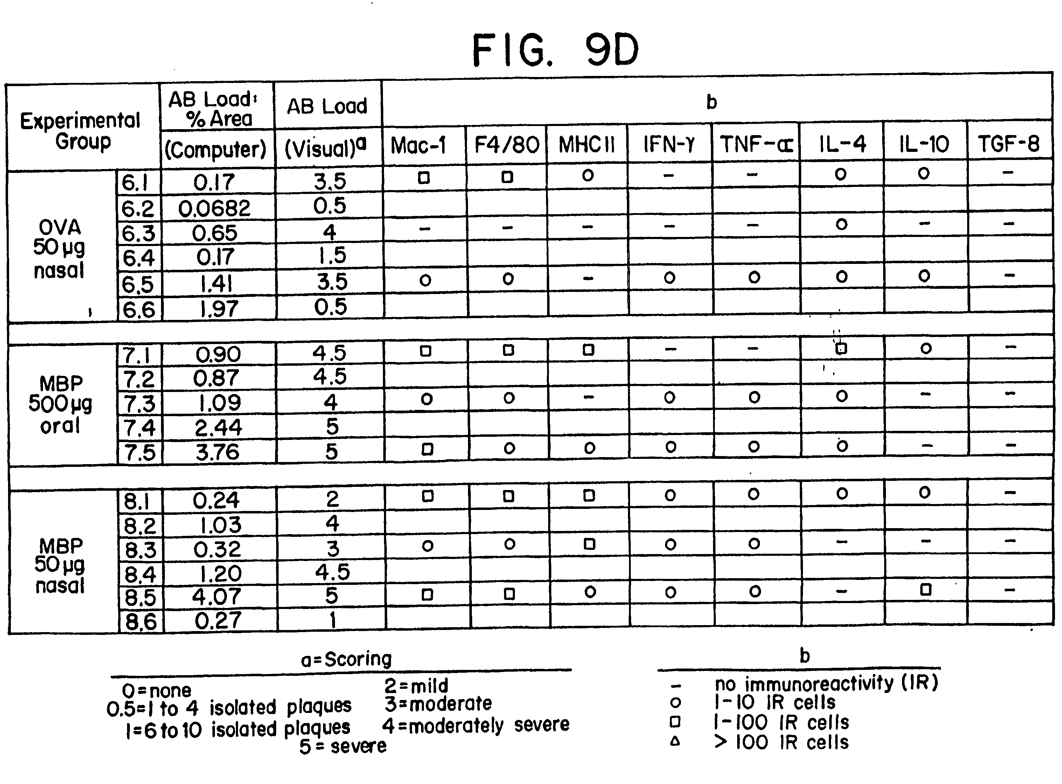

Results obtained in the examination of brain sections are shown in Figure 9A and Figure 9B. Specificially, results are shown for both visual and computer assisted image analysis of β-amyloid plaque load for the 8 experimental groups of mice that were tested; values are shown for each mouse in the relevant group. The computer analysis of plaque load represents the percent area identified as plaque. The visual assessment of plaque load was scored according to the table shown at the bottom of Figure 9A.

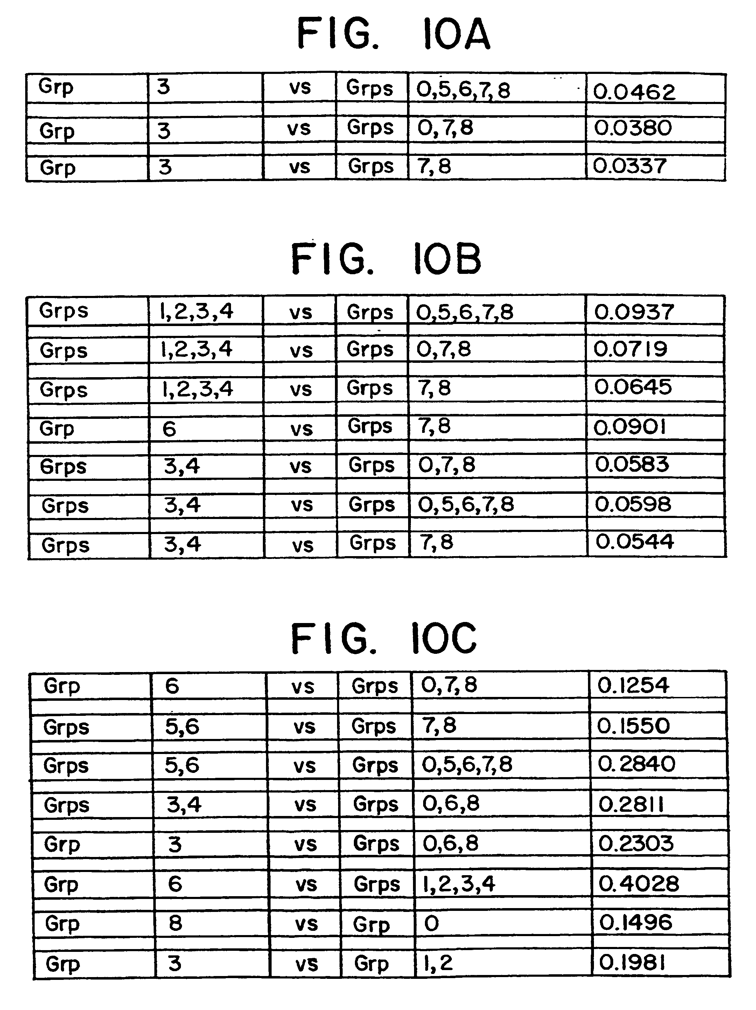

These results demonstrated a difference in the development of β-amyloid deposits in the mice treated with β-amyloid as opposed to the control groups, with the exception of certain ova treated groups, as discussed further below, i.e., the treated groups developed fewer β-amyloid deposits. The difference was statistically significant for mice that were nasally administered 25 µg of β-amyloid versus controls. P values calculated using the 1-tailed Mann-Whitney U-test for various group comparisons are shown in Figure 10.

Results of immunoreactivity tests conducted on the sections are also shown in Figure 9A and 9B, showing immunoreactivity (IR) of specific antibodies with cells bearing the immunological markers, or secreting the cytokines, shown. Results are shown in particular for the presence of: Mac-1, a marker for activated antigen presenting cells, such as microglia; F4/80, a marker for antigen presenting cells; and MHC-II. Results are also shown for immunoreactivity with the inflammatory cytokines IFN-γ, TFN-α, and for the anti-inflammatory cytokines IL-4, IL-10, and TGF-β. These data show that the mice treated mucosally with β-amyloid exhibited decreased microglial activation in the brain and decreased production of inflammatory cytokines.

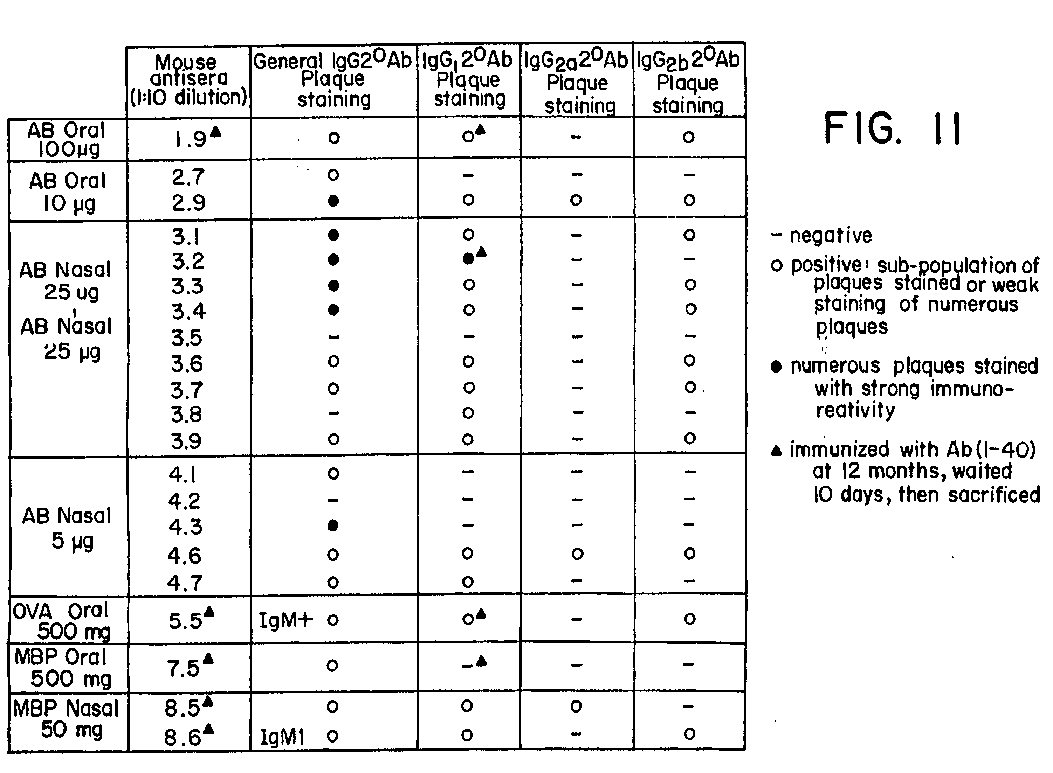

Results of immunohistochemistry experiments conducted on mouse antisera are shown in Figure 11. In these experiments, the antisera were tested in an immunoassay for the presence of IgG subtypes against β-amyloid. In the immunoassay, Down syndrome brain sections, which contain large amounts of β-amyloid, were employed as "capture" antigen. IgG in the mice antisera were bound to the sections, and the presence of IgG subtypes in the bound antibodies distinguished by secondary (2°) antibodies specific for each subtype. These results demonstrated an increase in IgG1 class antibodies against β-amyloid in antisera of mice that were nasally treated with 25 µg of β-amyloid over that observed in controls. These antibodies are associated with Th2 type cellular immune responses.

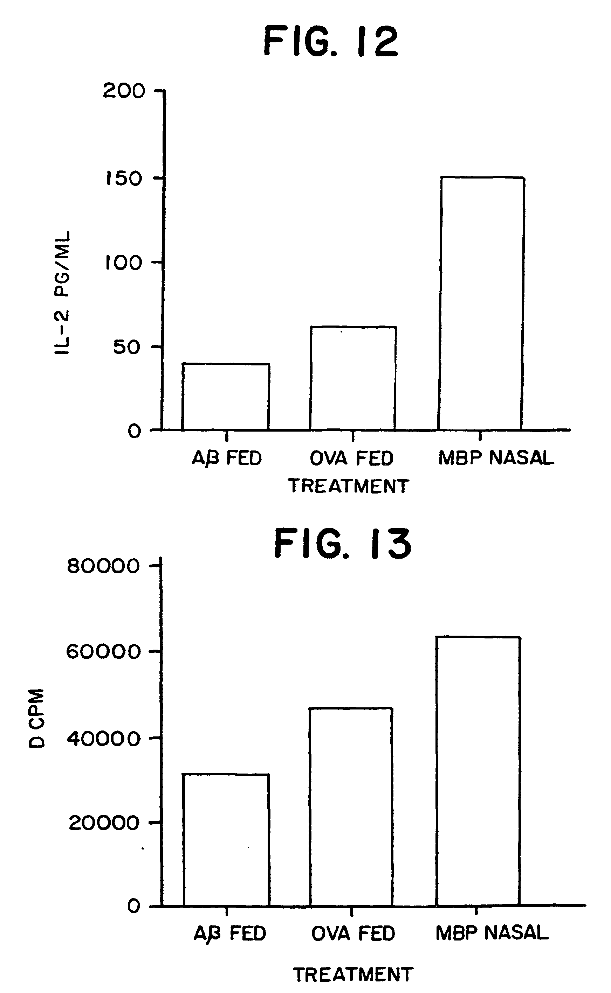

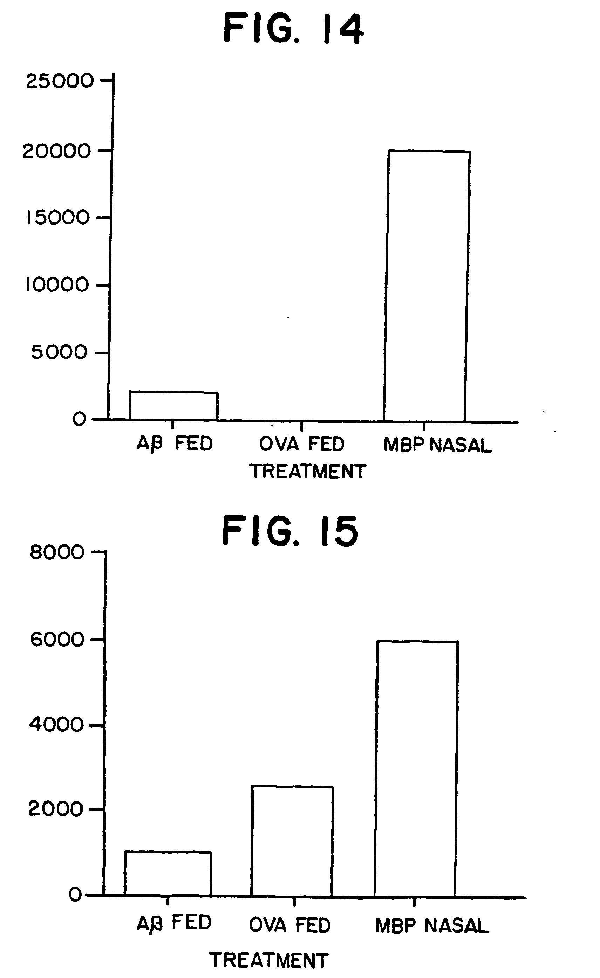

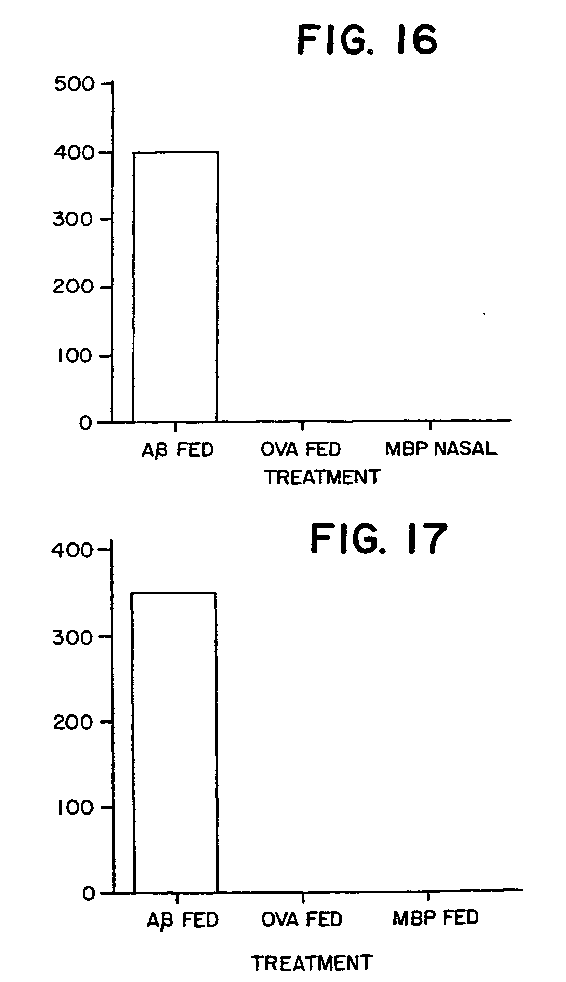

Figures 12-17 are bar graphs showing results of *in vitro* experiments in which PLN from the PDAPP mice that were immunized with [1-40] β-amyloid peptide prior to sacrifice were tested by stimulation with the peptide, and tested for cytokine production. In the animals that were mucosally administered the [1-40] β-amyloid peptide, PLN immune responses were characterized by increased production of IL-10 and TGF-β and decreased production of IFN-γ, IL-6, and IL-2.

In several of the experiments described above, results were obtained for administration of whole ovalbumin that were substantially different from those obtained using the other controls, and similar to results obtained using [1-40] β-amyloid. Further experiments were therefore conducted in which it was determined that whole ovalbumin is cross-reactive with [1-40] β-amyloid. Ovalbumin is therefore not an effective control in experiments designed to test immunological properties of [1-40] β-amyloid.

### Example 5

### Administration of β-amyloid In Conjunction with IL-4 or IL-10

Experiments were conducted in which [1-40] β-amyloid was nasally administered to (B6D2)F1 mice in conjunction with nasal administration of IL-4 or IL-10, and prior to immunization of the mice with [1-40] β-amyloid. Specifically, the (B6D2)F1 mice were nasally treated with the following compositions:
1) PBS;
2) [1-40] β-amyloid;
3) IL-4;
4) [1-40] β-amyloid+IL-4;
5) IL-10; or
6) [1-40] β-amyloid+IL-10

The [1-40] β-amyloid was administered in a dose of 25µg and the IL-4 and IL-10 administered in a dose of 1µg. The compositions were administered three times, at intervals of two days. Two days after the last treatment the mice were immunized with 100µg [1-40] β-amyloid in 50µg CFA. Two weeks later splenocytes from the mice were examined for proliferation and cytokine production in the presence of 50µg/ml [1-40] β-amyloid *in vitro.*

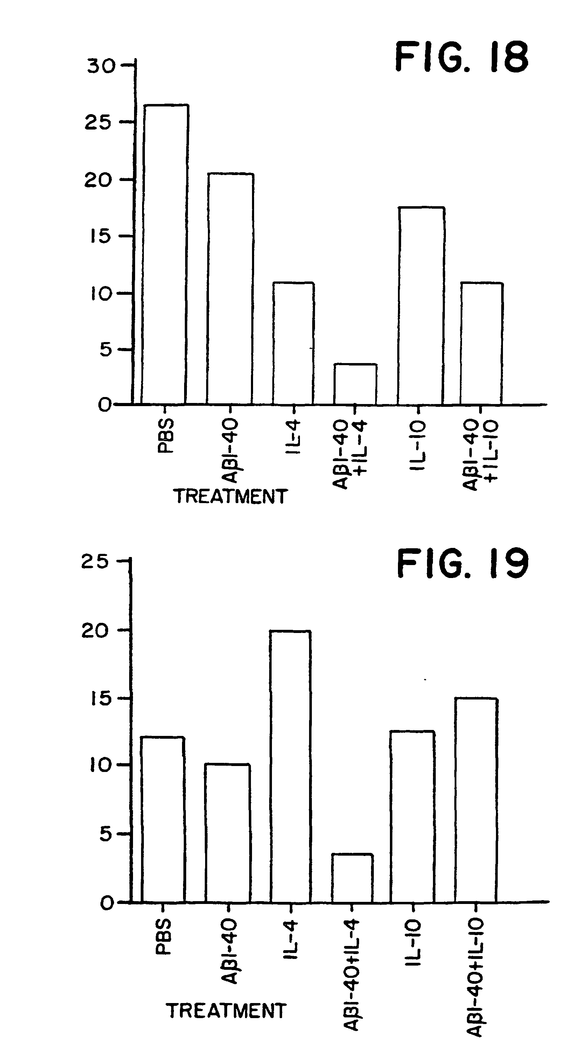

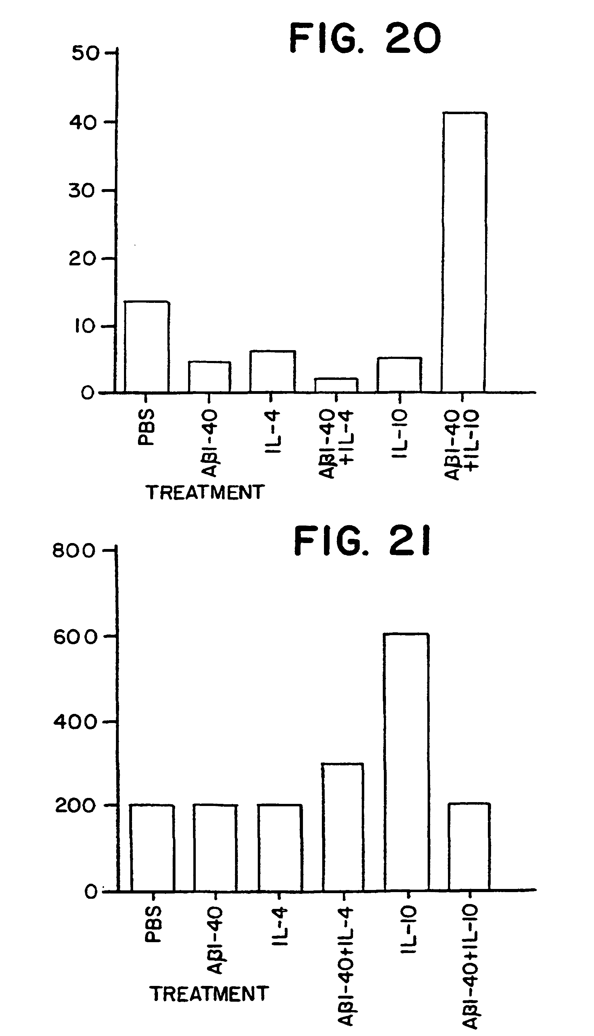

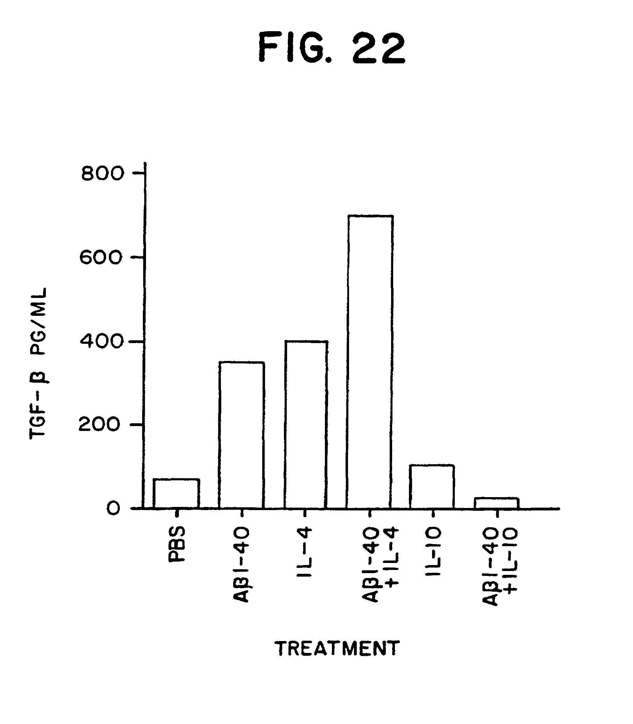

Results from these experiments are shown in Figures 18-22. In these experiments, administration of IL-4 was shown to be particularly effective in enhancing suppressive responses induced by β-amyloid peptide administration, as shown, for example in suppression of IFN-γ production, increased TGF-β production, and increased proliferative responses. The substantial decrease in IL-6 production induced by IL-4 administration in conjunction with β-amyloid peptide is relevant as increased IL-6 production is correlated with Alzheimer's disease.

### Example 6

### Human Treatment

An individual afflicted with Alzheimer's disease is first orally or nasally administered 0.05 mg of [1-40] β-amyloid peptide once a day for a period of one to two weeks at the end of which the patient's cytokine responses are measured using ELISA techniques as described above. If no improvement is observed, the daily dosage is increased (progressively) to 0.1 mg, 0.5 mg, 1 mg, 2.5 mg, 5 mg, 50mg or 100 mg, etc. (and the cytokine responses are monitored weekly or every two weeks) until an effective dosage is determined. (The number of daily doses may also be progressively increased up to six daily either instead of or in addition to an increase in total daily amount of antigen administered.) Once an effective amount and administration schedule has been identified for this patient (over the course of no more than several weeks or longer), therapy continues at this amount and schedule for as long as is beneficial. Periodically, tests of cognitive skill are administered to monitor progress.

### Example 7

### Prevention of Alzheimer's Disease in Humans

An individual is determined to be at risk for contracting Alzheimer's disease by examination of the individual's family history of the disease, and by analysis of magnetic resonance, positron emission tomography, SPECT gamma camera imaging, and/or other imaging of the brain in conjunction with suitable image enhancement agents presently available. In this manner, the presence of amyloid plaque formation in the brain is observed. In addition, the size of the hippocampus is measured and compared with hippocampal size typically seen in development of Alzheimer's disease. These measures provide a reasonable prognosis for increased risk of developing Alzheimer's disease.

An individual at risk for contracting Alzheimer's disease is first orally or nasally administered 0.05 mg of [1-40] β-amyloid peptide once a day for a period of one to two weeks at the end of which the patient's cytokine and antibody responses are measured using ELISA techniques as described above. If no beneficial effect is observed, the daily dosage is increased (progressively) to 0.1 mg, 0.5 mg, 1 mg, 2.5 mg, 5 mg, 50mg or 100 mg, etc. (and the responses are monitored weekly or every two weeks) until an effective dosage is determined. (The number of daily doses may also be progressively increased up to six daily either instead of or in addition to an increase in total daily amount of antigen administered.) Once an effective amount and administration schedule has been identified for this patient (over the course of no more than several weeks), therapy continues at this amount and schedule for at least 6 months. Periodically, tests of cognitive skill are administered to monitor symptoms of disease.

The invention has been described above by reference to preferred embodiments. It will be understood that various modifications are possible within the scope of the claims that follow. In case of conflict, the present disclosure including the present definitions will control.

## Claims

1. A pharmaceutical formulation adapted for nasal administration in an aerosol form to a mammal at risk of contracting Alzheimer's disease comprising an effective amount of a polypeptide comprising an amino acid sequence of β-amyloid peptide or an effective fragment or analog thereof for inhibiting or delaying the onset of at least one of the following:
(i) generation of one or more proinflammatory Th1 cytokines in the mammal;
(ii) decline in the quantity of one or more anti-inflammatory Th2 or Th3 cytokines in the mammal; or
(iii) appearance of at least one clinical symptom of the disease.

2. A pharmaceutical formulation as claimed in claim 1 further comprising a pharmaceutically acceptable carrier or diluent.

3. The pharmaceutical formulation of claim 1 or claim 2, wherein the dose is greater than 50 µg per day.

4. The pharmaceutical formulation of any one of claims 1 to 3, adapted for administration via a nebulizer, a pressurized metered dose inhaler or a dry powder inhaler.

5. The pharmaceutical formulation of any one of claims 1 to 4, formulated as a dry powder.

6. The pharmaceutical formulation of any one of claims 1 to 4, formulated as an aqueous solution.

7. The use of an effective amount of a polypeptide comprising an amino acid sequence of β-amyloid peptide or an effective fragment or analog thereof in the manufacture of a medicament for nasal administration in an aerosol form, for treating a mammal suffering from Alzheimer's disease wherein the medicament is to be administered for a period of time sufficient to achieve at least one of the following:
(i) reduce the quantity of one or more proinflammatory Th1 cytokines in the mammal;
(ii) increase the quantity of one or more anti-inflammatory Th2 or Th3 cytokines in the mammal; or
(iii) ameliorate, inhibit or delay the onset of at least one clinical symptom of the disease.

8. The use as claimed in claim 7 wherein the medicament is for administration by inhalation.

9. A use as claimed in claim 7 or claim 8 wherein the peptide is β-amyloid peptide, or wherein said peptide fragment comprises a homologous, conservative, non-peptidic or isosteric replacement for one or more amino acids of β-amyloid peptide.

10. The use of any one of claims 7 to 9, wherein the dose is greater than 50 µg per day.

11. The use of any one of claims 7 to 11, wherein the medicament is adapted for nasal administration via a nebulizer, a pressurized metered dose inhaler or a dry powder inhaler.

12. The use of any one of claims 7 to 11, wherein the medicament is a dry powder.

13. The use of any one of claims 7 to 11, wherein the medicament is an aqueous solution.

## Patentansprüche

1. Pharmazeutische Formulierung, die für eine nasale Verabreichung in einer Aerosolform an einen Säuger angepasst ist, der ein Risiko für eine Erkrankung an der Alzheimer-Krankheit aufweist, umfassend eine wirksame Menge eines Polypeptids, das eine Aminosäuresequenz von β-Amyloid-Peptid oder ein wirksames Fragment oder Analogon davon umfasst, die für die Hemmung oder Verzögerung des Einsetzens von wenigstens einer der nachstehenden Wirkungen ausreichend ist:
(i) Herstellung eines oder mehrerer proinflammatorischer Th1-Cytokine in dem Säuger;
(ii) Abnahme der Menge eines oder mehrerer entzündungshemmender Th2- oder Th3-Cytokine in dem Säuger; oder
(iii) Auftreten von wenigstens einem klinischen Symptom der Krankheit.

2. Pharmazeutische Formulierung gemäß Anspruch 1, ferner umfassend ein pharmazeutisch verträgliches Trägermaterial oder Verdünnungsmittel.

3. Pharmazeutische Formulierung gemäß Anspruch 1 oder Anspruch 2, wobei die Dosis größer als 50 µg pro Tag ist.

4. Pharmazeutische Formulierung gemäß einem der Ansprüche 1 bis 3, angepasst an die Verabreichung über einen Vernebler, Dosier-Kompressions-Inhalator oder einen Trocken-Pulver-Inhalator.

5. Pharmazeutische Formulierung gemäß einem der Ansprüche 1 bis 4, formuliert als trockenes Pulver.

6. Pharmazeutische Formulierung gemäß einem der Ansprüche 1 bis 4, formuliert als wässrige Lösung.

7. Verwendung einer wirksamen Menge eines Polypeptids, das eine Aminosäuresequenz von β-Amyloid-Peptid oder ein wirksames Fragment oder ein Analogon davon umfasst, zur Herstellung eines Medikaments zur nasalen Verabreichung in Aerosolform, zur Behandlung eines Säugers, der an der Alzheimer-Krankheit erkrankt ist, wobei das Medikament für eine Zeitdauer verabreicht wird, die ausreicht wenigstens eine der nachstehenden Wirkungen zur erreichen:
(i) Reduktion der Menge eines oder mehrerer proinflammatorischer Th1-Cytokine in dem Säuger;
(ii) Erhöhung der Menge eines oder mehrerer entzündungshemmender Th2- oder Th3-Cytokine in dem Säuger; oder
(iii) Linderung, Hemmung oder Verzögerung des Ausbruchs von wenigstens einem klinischem Symptom der Krankheit.

8. Verwendung gemäß Anspruch 7, wobei das Medikament zur Verabreichung durch Inhalation ist.

9. Verwendung gemäß Anspruch 7 oder Anspruch 8, wobei das Peptid ein β-Amyloid-Peptid ist, oder wobei dieses Peptid-Fragment einen homologen, konservativen, nichtpeptid oder isosterischen Austausch von einer oder mehreren Aminosäuren des β-Amyloid-Peptid umfasst.

10. Verwendung gemäß eines der Ansprüche 7 bis 9, wobei die Dosis größer als 50 µg pro Tag ist.

11. Verwendung gemäß eines der Ansprüche 7 bis 11, wobei das Medikament an die nasale Verabreichung über einen Vernebler, Dosier-Kompressions-Inhalator oder einen Trocken-Pulver-Inhalator angepasst ist.

12. Verwendung gemäß eines der Ansprüche 7 bis 11, wobei das Medikament trockenes Pulver ist.

13. Verwendung gemäß eines der Ansprüche 7 bis 11, wobei das Medikament eine wässrige Lösung ist.

## Revendications

1. Formulation pharmaceutique, sous forme d'aérosol, adaptée à l'administration nasale à un mammifère qui risque de contracter la maladie d'Alzheimer, comprenant une quantité efficace d'un polypeptide comprenant une séquence d'acides aminés de peptide β-amyloïde ou d'un fragment efficace ou d'un analogue de celui-ci pour empêcher ou retarder l'apparition d'au moins l'un des phénomènes suivants :
(i) la génération d'une ou plusieurs cytokines pro-inflammatoires Th1 chez le mammifère;
(ii) le déclin de la quantité d'une ou plusieurs cytokines anti-inflammatoires Th2 ou Th3 chez le mammifère; ou
(iii) l'apparition d'au moins un symptôme clinique de la maladie.

2. Formulation pharmaceutique selon la revendication 1, comprenant en outre un véhicule ou un diluant acceptable sur le plan pharmaceutique.

3. Formulation pharmaceutique selon la revendication 1 ou 2, dans laquelle la dose est supérieure à 50 microgrammes par jour.

4. Formulation pharmaceutique selon l'une quelconque des revendications 1 à 3, adaptée à une administration au moyen d'un nébuliseur, d'un inhalateur à dose mesurée sous pression ou d'un inhalateur à poudre sèche.

5. Formulation pharmaceutique selon l'une quelconque des revendications 1 à 4, formulée en poudre sèche.

6. Formulation pharmaceutique selon l'une quelconque des revendications 1 à 4, formulée en solution aqueuse.

7. Utilisation d'une quantité efficace d'un polypeptide comprenant une séquence d'acides aminés de peptide β-amyloïde ou d'un fragment efficace ou d'un analogue de celui-ci pour la fabrication d'un médicament pour administration nasale, sous forme d'aérosol, afin de traiter un mammifère souffrant de la maladie d'Alzheimer, dans laquelle le médicament doit être administré sur une période de temps suffisante pour obtenir au moins l'un des résultats suivants :
(i) la réduction de la quantité d'une ou plusieurs cytokines pro-inflammatoires Th1 chez le mammifère;
(ii) l'augmentation de la quantité d'une ou plusieurs cytokines anti-inflammatoires Th2 ou Th3 chez le mammifère; ou
(iii) l'amélioration, la prévention ou le retardement de l'apparition d'au moins un symptôme clinique de la maladie.

8. Utilisation selon la revendication 7, dans laquelle le médicament est destiné à une administration par inhalation.

9. Utilisation selon la revendication 7 ou 8, dans laquelle le peptide est un peptide β-amyloïde ou dans laquelle ledit fragment de peptide comprend un substitut homologue, conservateur, non peptidique ou isostère pour un ou plusieurs acides aminés du peptide β-amyloïde.

10. Utilisation selon l'une quelconque des revendications 7 à 9, dans laquelle la dose est supérieure à 50 microgrammes par jour.

11. Utilisation selon l'une quelconque des revendications 7 à 11, dans laquelle le médicament est à même d'être utilisé pour administration nasale au moyen d'un nébuliseur, d'un inhalateur à dose mesurée sous pression ou d'un inhalateur à poudre sèche.

12. Utilisation selon l'une quelconque des revendications 7 à 11, dans laquelle le médicament est une poudre sèche.

13. Utilisation selon l'une quelconque des revendications 7 à 11, dans laquelle le médicament est une solution aqueuse.
